# EUROPEAN PATENT SPECIFICATION

(11) **EP 4 157 390 B1**
(45) Date of publication and mention of the grant of the patent: **30.07.2025**
(21) Application number: 21733724.5
(22) Date of filing: 01.06.2021
(51) Int. Cl.: A61M 1/16, A61M 1/36

(54) **SYSTEMS, SET AND DIALYSIS FLUID FOR EXTRACORPOREAL BLOOD TREATMENT**
SYSTEMEN, SET UND DIALYSIERFLÜSSIGKEIT ZUR EXTRAKORPORALEN BLUTBEHANDLUNG
SYSTEMES, SET ET LIQUIDE DE DIALYSE POUR LE TRAITEMENT EXTRACORPOREL DU SANG

(30) Priority: 01.06.2020 SE 2050621
(43) Date of publication of application: 05.04.2023
(73) Proprietor: Gambro Lundia AB, 226 43 Lund (SE)
(72) Inventor: HOBRO, Sture, 226 47 Lund (SE); FORSAL, Innas, 214 45 Malmö (SE); HANCOCK, Viktoria, 241 38 Eslöv (SE); NILSSON, Anders, 247 35 Södra Sandby (SE)
(74) Representative: Sweden SHS IP Office
(86) International application number: PCT/EP2021/064592
(87) International publication number: WO 2021/245040

(56) References cited:
- WO-A2-2011/070527
- US-A- 5 091 094
- US-A- 5 441 636

## Description

The disclosure herein relates to extracorporeal blood treatment. More particularly, the disclosure relates to the use of extracorporeal blood treatments such as hemodialysis in the treatment of cancer and to systems for such treatments and to user interfaces for the control of and the display of data recorded (s-g historical data) during such extracorporeal blood treatments.

Most human cancer cells show an altered energy metabolism that distinguishes them from normal cells. Normal cells acquire most of their energy through mitochondrial oxidative phosphorylation, an aerobic process in which glucose is oxidized, firstly through the glycolysis and subsequently through the tricarboxylic acid (TCA) cycle to produce adenosine triphosphate. This pathway is, on the contrary, only secondary in cancer cells. This was first observed in the 1920s by Warburg. He noted that after cancer cells have metabolized glucose by the glycolysis, lactate is produced from puruvate. In normal cells, this only occurs under anaerobic conditions, but in cancer cells, this alternative pathway is increased even in the presence of abundant oxygen. This phenomenon was named as "aerobic glycolysis" or "Warburg effect" (Warburg et al., 1927, Gen Physiol 8: 519-530). The presence of a characteristic glycolytic phenotype in cancer cells was confirmed by subsequent studies that have also observed overexpression of enzymes involved in glycolysis in most of cancer cells.

The above metabolic transformation confers to cancer cells a selective growth advantage and contributes to the ability to resist to hypoxia and apoptosis. Since the rate of tumor cell proliferation exceeds the rate of new blood vessel formation, many tumors grow in a low-oxygen environment. Various metabolic alterations in cancer cells exist and the most common and the most well-known is their habit to produce energy through aerobic glycolysis. Furthermore, many intermediates of glycolysis, such as, for example, ribose, glycerol and serine, are also intermediates of biosynthetic and anabolic pathways that are essential during cancer cell growth and proliferation. Also, glycolysis produces ATP from ADP which allows to sustain cell growth in the tumor. However, glycolysis is much less efficient than oxidative phosphorylation, and therefore requires a high amount of glucose to produce sufficient amounts of ATP. Therefore, this metabolic pathway requires a high amount of glucose. Many cancer cells become addicted to glucose as their main energy supplier. Owing to multiple reasons, glycolytic tumor cells become vulnerable if their glucose supply is targeted.

Further, many cancer cells also display addiction to glutamine. The high rate of glutamine uptake exhibited by glutamine-dependent cells is not only a result from its role as a nitrogen source in nucleotide and amino acid biosynthesis, but also, glutamine is the primary mitochondrial substrate in cancer and is required to produce NADPH for redox control and macromolecular synthesis.

Other metabolic alterations exist in cancers that have an important role in survival. Many metabolic alterations exist in cancers and various amino acids such as glutamine have important roles in cancer metabolism to control redox balance and to produce building blocks for continued proliferation and further, many cancers show a surprisingly good ability to utilize those alternative pathways to change their metabolic profile when needed to adopt to new metabolic limitations. This quality (ability) is an important feature for cancers and their ability to withstand environmental challenges when metabolic energy resources such as glucose, glutamine, or oxygen become scarce.

Therefore, to effectively affect cancer through a metabolic approach it is important to affect their metabolic system from more than one direction. Reduced glucose can by most cancer cells be readily handled, but if several metabolic possibilities are affected at the same time (as reducing glucose and glutamine) the sum of these changes becomes much more devastating than the individual parts.

Beside glucose, ketone and glutamine, which are global energy sources for many cancers, serine and glycine meet important specific needs to sustain cell growth and proliferation in cancer, for example through the one-carbon metabolism. In addition to a large energy requirement, cancer cells must also accumulate building blocks for the construction of new cellular components, including nucleic acids, proteins, and lipids, as well as equally important cofactors for the maintenance of their cellular redox status (Amelio et al.: Trends. Biochem.Sci. (2014), vol. 39(4): 191-198)).

Studies have demonstrated that arginine is necessary for cellular growth and can become limiting in states of rapid growth and if deprived from the cancer cells also effect survival (Albaugh et al., J.Surg. Oncol. (2017), vol. 115(3), 273-280).

In view of the above, it has been suggested that reduced glycaemia may serve as a strategy to target a broad range of glycolysis dependent tumors. In low glycaemia conditions, fats and especially ketone bodies can replace glucose as a primary metabolic fuel for normal cells. Many tumors, however, have abnormalities in the genes and enzymes needed to metabolize lipids and ketone bodies for energy. Therefore, a transition from carbohydrate to ketones for energy specifically targets the energy metabolism in glycolysis-dependent tumor cells (Seyfried et al, 2010, Nutrition and Metabolism, 7:7).

In accordance with this approach, for example, WO2011070527 discloses a method of treatment of a proliferative disorder, cancerous or non-cancerous, in an individual wherein a hemodialysis apparatus is used to reduce blood glucose concentration.

The use of a hemodialysis apparatus for reducing glycemia has the advantage that the glucose concentration in the blood can be reduced and thereby decreased in a more controlled and effective way compared to diet glucose deprivation. However, the method and apparatus disclosed in WO2011070527 require blood glucose sensors and blood glutamine sensors connected to the blood intake-flow, the blood return flow and the dialysate, which sensors all are connected to the central control unit of the hemodialysis machine. Moreover, the central control unit of WO2011070527 is also connected to an electroencephalograph (EEG) in order to provide the central unit with information pertaining to spontaneous electro-cerebral activity to initiate raising of glucose and glutamine levels. Such a large number of sensors and instruments leads to a high level of complexity and associated high cost. Further, patients undergoing this treatment must consume only a glucose restricted diet for several days prior to undertaking the treatment. This is not an insignificant burden on the patient.

There is a constant need for improved ways of treating cancer.

US, A, 5,091,094 discloses techniques for predicting the respective concentrations and distributions of diffusible materials in solutions on opposing sides of a semipermeable membrane. Unique compositions for hemodialysis using the mathematical relationships involved are provided.

### SUMMARY

In a first aspect of the invention, there is provided an extracorporeal blood treatment system (50) for treating a subject suffering from cancer, the system comprising:
an extracorporeal blood circuit (52a, 52b);
a dialysate fluid circuit (54a, 54b);
said extracorporeal blood circuit (52a, 52b) and dialysate fluid circuit (54a, 54b) being divided by a membrane (59) of a filtration unit (58);
at least one blood pump (60) for controlling the flow of blood through the blood circuit (52a, 52b);
at least one dialysate fluid pump (62, 68) for controlling the flow of dialysate fluid through the dialysate fluid circuit (54a, 54b);
optionally one or more infusion lines (66, 80, 81, 82), each infusion line being connected to the extracorporeal blood circuit (52a, 52b) or being adapted to be directly connected to the vascular system of the subject to be treated, each infusion line comprising an infusion pump;
a system computing unit (64) operatively connected to the blood pump (60) and the dialysate fluid pump (62, 68) and optionally to the one or more infusion pumps of the one or more infusion lines (66, 80, 81, 82), said system computing unit having a user interface including an input means and a display means; wherein
the system computing unit (64) is adapted for receiving a desired blood concentration value GLN_{b} of glutamine (901) within the range of 0.1 and 0.5 mM;
the system computing unit (64) is adapted for receiving a desired blood concentration value GLUCOSE_{b} of glucose (903) within the range of 2 and 4 mM;
the system computing unit (64) is adapted for receiving a desired blood concentration value KETONE_{b} of a ketone body such as acetoacetate, beta-hydroxybutyrate or pharmaceutically acceptable derivatives, esters, and salts thereof (905) within the range of 1 - 15 mM;
the system computing unit (64) is adapted for receiving a concentration value GLNₚ representing the concentration of glutamine or pharmaceutically acceptable glutamine-containing compounds in fresh dialysate fluid (902);
the system computing unit (64) is adapted for receiving a concentration value GLUCOSEₚ representing the concentration of glucose in fresh dialysate fluid ₍904); optionally, the system computing unit (64) is adapted for receiving a concentration value KETONEₚ,,representing the concentration of a ketone body, such as acetoacetate, beta-hydroxybutyrate or pharmaceutically acceptable derivatives, esters and salts thereof in fresh dialysate fluid (907);
optionally, the system computing unit (64) is adapted for receiving a concentration value KETONEᵢ representing the concentration of a ketone body, such as acetoacetate, beta-hydroxybutyrate or pharmaceutically acceptable derivatives, esters and salts thereof in an infusion liquid to be infused into the extracorporeal blood line (52b) or directly into the vascular system of said subject to be treated through one of said one or more infusion lines (66, 80, 81, 82) (906);
the system computing unit (64) is adapted for receiving an actual concentration value GLNₐ of glutamine (909) in blood from said treated subject, and receiving an actual blood concentration value GLUCOSEₐ of glucose (910) in blood from said treated subject, and receiving an actual concentration value KETONEₐ of a ketone body such as acetoacetate and beta-hydroxybutyrate (911);
the system computing unit (64) being adapted for controlling said blood pump (60) and said dialysate fluid pump (62, 68) in such a way that the actual blood concentration value GLNₐ of glutamine is driven towards or below GLN_{b} (912) and the actual blood concentration value GLUCOSEₐ of glucose is driven towards or below GLUCOSE_{b} (914);
   and
if the system (50) comprises one or more of said infusion lines (66, 80, 81, 82) and in case one of said infusion lines (66, 80, 81, 82) is adapted for infusing said infusion liquid into the extracorporeal bloodline (52b) or directly into the vascular system of said subject to be treated, the system computing unit (64) is adapted for controlling said infusion pump of said infusion line in such a way that the actual blood concentration value KETONEₐ is driven towards KETONE_{b} (920);
or alternatively
if the system (50) does not comprise such an infusion line (66, 80, 81, 82), the system computing unit (64) is adapted for comparing KETONEₐ and KETONE_{b}, and if KETONEₐ < KETONE_{b}, displaying a message on said display informing that the treated subject should consume a further amount of ketone bodies or medium chain triglycerides.

In this disclosure the term "subject" relates to a human or animal patient in need of treatment.

In a preferred embodiment, the extracorporeal blood treatment system (50) comprises one or more of said infusion lines (66, 80, 81, 82), and the system computing unit (64) is adapted for receiving a concentration value, KETONEᵢ, representing the concentration of a ketone body such as acetoacetate, beta-hydroxybutyrate, or pharmaceutically acceptable derivatives, esters and salts thereof in an infusion liquid to be infused into the extracoprporeal blood line (52b) or directly into the vascular system of the subject to be treated, through one of said one or more infusion lines (66, 80, 81, 82) (906), and
the system computing unit (64) is adapted for controlling said infusion pump of said infusion line in such a way that the actual blood concentration value KETONEₐ is driven towards KETONE_{b} (920).

In a preferred embodiment the system computing unit (64) is adapted for monitoring GLNₐ , and GLUCOSEₐ,_{,} and initiating infusion of a composition containing glutamine or pharmaceutically acceptable glutamine-containing compounds if GLNₐ is lower than GLN_{b} (916) and/or initiating infusion of a composition containing glucose if GLUCOSEₐ is lower than GLUCOSE_{b} (918) by starting a relevant infusion pump in one of the one or more infusion lines (66, 80, 81, 82), and maintaining said infusion until GLNₐ is equal to GLN_{b} and GLUCOSEₐ is equal to GLUCOSE_{b}.

The term "pharmaceutically acceptable glutamine-containing compounds" relates to oligopeptides, typically dipeptides where at least one of the amino acid residues is glutamine. Typical examples of such dipeptides are L-alanyl-L-glutamine, and L-glycyl-L-glutamine. Glutamine-containing compounds are typically used instead of glutamine in liquid compositions in order to enhance stability and solubility.

The term "ketone bodies" relates to water-soluble molecules containing the ketone group that may be produced by the liver from fatty acids. Typically, a ketone body in accordance with the present invention is beta-hydroxybuturate or a pharmaceutically acceptable derivative thereof, such as its enantiomer (R)-beta-hydroxybutyric acid, (S)-beta-hydroxybutyrate, or enantiomeric mixture, or a pharmaceutically acceptable salt thereof, or a pharmaceutically acceptable ester thereof, as well as acetoacetate. Medium chain triglycerides are also considered to be derivatives of a ketone body in accordance with the present invention. The term "medium chain triglycerides" or "MCT oils" are triglycerides with two or three fatty acids having an aliphatic tail of 6 - 12 carbon atoms. Such medium chain triglycerides or MCT oils may be transformed into ketone bodies in the human body. Examples of infusion liquids containing ketone bodies or ketone body derivatives are Lipofundin ^{®} MCT/LCT 20 % (B. Braun) or SMOFlipid ^{®} 20 % (Fresenius Kabi). Further examples can be found in WO2018/114309 A1. Only the concentration of beta-hydroxybutyrate and/or acetoacetate are detected in the blood of a subject as the actual ketone body concentration KETONEₐ as the other ketone body derivatives are transformed into any of these compounds in the body of the subject.

Preferably, the filtration membrane has a molecular weight cut off (MWCO) of less than 60kDa.

More preferably, the filtration membrane has a MWCO of less than about 50kDa or less than about 40 kDa (such as less than 30 kDa, less than 10 kDa, less than 5 kDa or less than 2 kDa). Preferably, the blood circuit comprises a thermal management system for heating or cooling blood in the blood line during use.

More preferably, the thermal management system is controllable to regulate the temperature of blood in the blood circuit to a temperature between 20°C and 43°C.

Preferably, the system (50) further comprises one or more sensors (S, 90, 91, 92, 93) for the detection of analytes selected from the group glucose, glutamine, and ketone bodies, the sensor(s) (S, 90, 91, 92, 93) being preferably positioned in an effluent portion (54a) of the dialysate fluid circuit, the sensors being in communication with the system processing unit (64) and providing an output indicative of the concentration of the analyte(s) in blood or preferably in spent dialysate fluid; wherein the system processing unit (64) is configured to determine from the output(s) of the sensor(s) (S, 90, 91, 92, 93) a representative blood concentration of the analyte(s), thereby monitoring at least one, and preferably all of GLNₐ, GLUCOSEₐ and KETONEₐ.

In a second aspect, the present invention provides a therapy set for use in an extracorporeal blood treatment system (50) according to the first aspect, the set comprising a filtration unit (58) having a membrane (59) which divides an integrated blood line (52a, 52b) and an integrated dialysate fluid line (54a, 54b), wherein the blood line (52a, 52b) and/or the dialysate fluid line (54a, 54b) comprises sensors (S, 90, 91, 92, 93) for monitoring at least one of GLNₐ, GLUCOSEₐ, and KETONEₐ.

As is shown below, suitable sensors and assays for analysing glutamine, glucose and ketone bodies are known in the art.

In a third aspect, which does not form part of the claimed invention, is disclosed a method of treating a subject suffering from cancer using a system (50) comprising:
an extracorporeal blood circuit (52a, 52b);
a dialysate fluid circuit (54a, 54b);
said extracorporeal blood circuit (52a, 52b) and dialysate fluid circuit (54a, 54b) being divided by a membrane (59) of a filtration unit (58);
at least one blood pump (60) for controlling the flow of blood through the blood circuit (52a, 52b);
at least one dialysate fluid pump (62, 68) for controlling the flow of dialysate fluid through the dialysate fluid circuit (54a, 54b);
and optionally one or more infusion lines (66, 80, 81, 82), each infusion line being connected to the extracorporeal circuit (52a, 52b) or adapted to be directly connected to the vascular system of a subject to be treated, each infusion line comprising an infusion pump;
wherein the method comprises:
   receiving a concentration value GLN_{b} representing a desired blood concentration of glutamine (901) within the range of 0.1 and 0.5 mM;
   receiving a concentration value GLUCOSE_{b} representing a desired blood concentration of glucose (903) within the range of 2 and 4 mM;
   receiving a concentration value KETONE_{b} representing a desired blood concentration of a ketone body such as acetoacetate, beta-hydroxybutyrate or pharmaceutically acceptable salts, derivatives and esters thereof (905) within the range of 1 and 15 mM; receiving a concentration value GLNₚ representing the concentration of glutamine or pharmaceutically acceptable glutamine-containing compounds in the fresh dialysate fluid (902);
   receiving a concentration value GLUCOSEₚ representing the concentration of glucose in the dialysate fluid (904);
   Optionally, the system computing unit
   optionally receiving a concentration value KETONEₚ,,representing the concentration of a ketone body, such as acetoacetate, beta-hydroxybutyrate or pharmaceutically acceptable derivatives, esters and salts thereof in fresh dialysate fluid (907);
   optionally receiving a concentration value KETONEᵢ representing the concentration of a ketone body, such as acetoacetate, beta-hydroxybutyrate or pharmaceutically acceptable salts, derivatives and esters thereof, (906) in an infusion liquid to be infused into the extracorporeal blood line (52b) or directly into the vascular system of said subject to be treated through one of said one or more infusion lines (66, 80, 81, 82);
   receiving a concentration value GLNₐ representing the actual concentration of glutamine in the blood of said treated subject (909);
   receiving a concentration value GLUCOSEₐ representing the actual concentration of glucose in the blood of said treated subject (910);
   receiving a concentration value KETONEₐ representing the actual concentration of a ketone body, such as acetoacetate and beta-hydroxybutyrate in the blood of said treated subject (911);
   controlling said blood pump (60) and said at least one dialysate fluid pump (62, 68) so that the actual concentration value GLNₐ of glutamine is driven towards or below GLN_{b} (912) and the actual concentration value GLUCOSEₐ of glucose is driven towards or below GLUCOSE_{b} (914);
      and
   if the system (50) comprises one or more of said infusion lines (66, 80, 81, 82) and one of said infusion lines (66, 80, 81, 82) infuses said infusion liquid into the extracorporeal blood line (52b) or directly into the vascular system of the subject to be treated, controlling said infusion in such a way that KETONEₐ is driven towards KETONE_{b} (920;
   or, alternatively
   if the system (50) does not infuse any infusion liquid containing a ketone body, the subject to be treated is asked to orally consume an amount of ketone bodies or medium chain triglycerides in case KETONEₐ is lower than KETONE_{b}.

Preferably, said system (50) comprises one or more of said infusion lines (66, 80, 81, 82), and the method further comprises:
receiving a concentration value KETONEᵢ representing the concentration of a ketone body such as acetoacetate, beta-hydroxybutyrate or pharmaceutically acceptable derivates, esters and salts thereof in an infusion liquid (906);
infusing said infusion liquid through one of said one or more infusion lines (66,80,81,82) into the extracorporeal blood line (52b) or directly into the vascular system of the subject to be treated; and
controlling the infusion pump of said infusion line in such a way that the actual blood concentration value KETONEₐ is driven towards KETONE_{b} (920).

Preferably, the cancer is selected from human colon carcinoma and glioblastoma, as well prostate, breast and liver cancer.

In a fourth aspect, the present invention provides a system computing unit (64) adapted for controlling an extracorporeal blood treatment system (50) for treating a subject suffering from cancer; said system computing unit comprising:
a plurality of output means adapted for being operatively connected to at least one blood pump (60), at least one dialysate fluid pump (62, 68) and optionally one or more infusion pumps for controlling the flow in each of one or more infusion lines (66, 80, 81, 82);
a user interface including an input means, and a display means; and
a memory means and a calculation means;
the system computing unit (64) being adapted for receiving a desired blood concentration value GLN_{b} of glutamine (901);
the system computing unit (64) being adapted for receiving a desired blood concentration value GLUCOSE_{b} of glucose (903);
the system computing unit (64) being adapted for receiving a desired blood concentration value KETONE_{b} of a ketone body (905)
the system computing unit (64) being adapted for receiving a dialysate concentration value GLNₚ of glutamine (902);
the system computing unit (64) being adapted for receiving a dialysate concentration value GLUCOSEₚ of glucose (904);
optionally, the system computing unit (64) is adapted for receiving a concentration value KETONEₚ,,representing the concentration of a ketone body, such as acetoacetate, beta-hydroxybutyrate or pharmaceutically acceptable derivatives, esters and salts thereof in fresh dialysate fluid (907);
optionally, the system computing unit (64) being adapted for receiving an infusion liquid concentration value KETONEᵢ of a ketone body (906);
the system computing unit (64) being adapted for receiving an actual blood concentration value GLNₐ of glutamine (909);
the system computing unit (64) being adapted for receiving an actual blood concentration value GLUCOSEₐ of glucose (910);
the system computing unit (64) being adapted for receiving an actual blood concentration value KETONEₐ of a ketone body (911) selected from the group of beta-hydroxybutyrate and acetoacetate;
the system computing unit (64) being adapted for controlling said blood pump (60) and said dialysate fluid pump (62, 68) in such a way that the actual blood concentration value GLNₐ of glutamine is driven towards or below GLN_{b} (912), and the actual blood concentration value GLUCOSEₐ of glucose is driven towards or below Glucose_{b} (914);
   and
in case an infusion pump is operatively connected to the system computing unit (64), the system computing unit (64) is adapted for controlling the infusion pump in such a way that the actual blood concentration value KETONEₐ is driven towards KETONE_{b} (920);
   and
in case no infusion pump is operatively connected to the system computing unit (64), the system computing unit (64) is adapted for comparing KETONEₐ and KETONE_{b}, and if KETONEₐ < KETONE_{b}, displaying a message on said display informing that the treated subject should consume a further amount of ketone bodies or medium chain triglycerides.

In a fifth aspect, the present invention provides a dialysis fluid comprising ketone bodies, such as acetoacetate, beta-hydroxybutyrate or pharmaceutically acceptable derivatives, esters and salts thereof, for use in a dialysis therapy method of treating cancer.

Preferably, the dialysis fluid also comprises a) glutamine or glutamine-containing compounds; and/or b) glucose.

Preferably, the concentration of:
a) glutamine or glutamine-containing compounds amounts to 0 - 0.5 mM, and more preferably 0.05 - 0.3 mM;
b) glucose amounts to 0 - 6 mM and more preferably 0.5 - 4 mM; and
c) ketone bodies amount to 1 - 15 mM and more preferably 2 - 12 mM.

The above summary of the present disclosure is not intended to describe each embodiment or every implementation thereof. Advantages, together with a more complete understanding of the present disclosure, will become apparent and appreciated by referring to the following detailed description and claims taken in conjunction with the accompanying drawings.

### BRIEF DESCRIPTION OF THE DRAWINGS

FIG. 1 is a schematic diagram of a system according to the present invention while in use.
FIG. 2 is a block diagram of an exemplary extracorporeal blood treatment system including input apparatus and display apparatus that may utilize the user interfaces and methods described herein.
FIG. 3 is a perspective illustration of an exemplary dialysis system that may include a graphical user interface as described herein.
FIG. 4 is a front view of a portion of the exemplary dialysis system shown in FIG. 2.
FIG. 5 is a schematic view of a part of a system having thermal management according to an embodiment of the present invention.
FIG. 6 shows charts of the results of Study 1.
FIG. 7 shows charts of the results of Study 2.
FIG. 8A, 8B and 8C illustrate the treatment goals in accordance with the present invention.
FIG. 9 is a flow chart showing the control algorithm of the present invention.
FIG. 10 shows growth rate of A549 and RCC4 cells in Medium A-C as indicated.
FIG. 11 presents amounts of lung carcinoma A549, renal carcinoma RCC4 and primary RCC cells after 3 days culture in Medium A-C at 21 or 5% oxygen. Stars denotes significantly different values determined by 2way ANOVA with Tukey's multiple comparison test.
FIG. 12 shows results of culture of human glioma cell line A172 at normoxia and hypoxia in Medium A-C with the addition of 8 mM Acac, 16 mM BOHB or the combination of 4 mM Acac / 8 mM BOHB. 4 and 8 mM LiCl are used as controls for Acac. Significantly different values determined by one-way ANOVA with Sidak's multiple comparisons test are marked with stars.
FIG. 13 illustrates results of culture of glioma cell line U118MG at normoxia and hypoxia in Medium A-C with the addition of 8 mM Acac, 16 mM BOHB or the combination of 4 mM Acac / 8 mM BOHB. 4 and 8 mM LiCl are used as controls for Acac. Significantly different values determined by one-way ANOVA with Sidak's multiple comparisons test are marked with stars.
FIG. 14 presents results of culture of glioma cell line A172 at normoxia and hypoxia in Medium B-C with the addition of 8 mM Acac, 16 mM BOHB or the combination of 4 mM Acac and 8 mM BOHB. 4 and 8 mM LiCl are used as controls for the 4 mM Acac/8 mM BOHB or 8 mM Acac, respectively. Significantly different values determined by one-way ANOVA with Sidak's multiple comparisons test are marked with stars.
FIG. 15 presents results of culture of glioma cell line U118MG at normoxia and hypoxia in Medium B-C with the addition of 8 mM Acac, 16 mM BOHB or the combination of 4 mM Acac and 8 mM BOHB. 4 and 8 mM LiCl are used as controls for the 4 mM Acac/8 mM BOHB or 8 mM Acac, respectively. Significantly different values determined by one-way ANOVA with Sidak's multiple comparisons test are marked with stars.
FIG. 16 presents results of culture of renal carcinoma cell line RCC4 at normoxia and hypoxia in Medium B-C with the addition of 8 mM Acac, 16 mM BOHB or the combination of 4 mM Acac and 8 mM BOHB. 4 and 8 mM LiCl are used as controls for the 4 mM Acac/8 mM BOHB or 8 mM Acac, respectively. Significantly different values determined by one-way ANOVA with Sidak's multiple comparisons test are marked with stars.

### DETAILED DESCRIPTION OF EXEMPLARY EMBODIMENTS

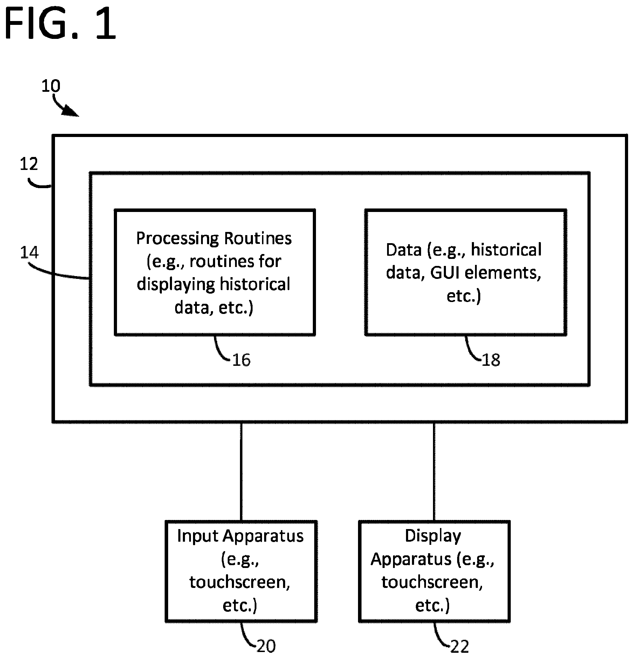

Exemplary systems and methods of selecting, viewing, and filtering historical data for extracorporeal blood treatments shall be described with reference to Figures 1-5. Extracorporeal blood treatment systems may log, or store, one or more parameters and/or events of one or more extracorporeal blood treatments resulting in historical data. The exemplary systems and methods described herein provide graphical user interfaces to display such historical data. Generally, the historical data may include patient fluid removal data, fluids data, treatment data, anticoagulation data, pressure data, event data, settings data, patient data, alarm data, system voltage and current data, system timing data, user interaction data (e.g., interactions with the user interface such as button presses and screen selection), etc. In the present disclosure, the data may include the concentration of certain substances in the patient's blood stream as the system may comprise one or more sensors for detecting those concentrations. The detected substances may include glucose, glutamine and other amino acids such as serine, glycine and arginine, ketones and cytokines.

The patient fluid removal data may include total patient fluid removal data, unintended patient fluid removal data, selected limit data (e.g., selected limits for unintended patient fluid gain/loss over a selected period such as 1 hour, 3 hours, or 24 hours), etc. The fluids data may include pre-infusion data, dialysate data, post-replacement fluid post filter data, filter data, effluent data, filtration fraction data, predilution data, rate per patient kilogram data, ultrafiltration rate data, ultrafiltration rate post % of blood flow rate data, etc. The treatment data may include prescribed effluent dose data, delivered effluent dose data, target effluent dose data, prescribed ultrafiltration rate (UFR) dose data, target UFR dose data, delivered UFR dose data, etc. The anticoagulation data may include heparin data, estimated patient citrate load data, citrate solution data, calcium solution data, replacement solution data, calcium compensation data, syringe volume delivery data, bolus delivery data, etc. The pressure data may include access line pressure data, return line pressure data, filter pressure data, transmembrane pressure (TMP) data, pressure drop across the filter (P DROP) data (e.g., the pressure conditions in the blood compartment of a filter), self-test data, pressure alarm data, disconnect and occlusion limit data, stabilization pressure data, etc. The event data may include system configuration data, alarm data, settings data, therapy set data, advisory data, prescription settings data, system settings data, anticoagulation data, pressure data, patient data, mechanical data, dose data, etc.

An exemplary extracorporeal blood treatment system 10 depicted in FIG. 1 may be used to execute the exemplary methods and/or processes described herein. In at least one embodiment, the system 10 may be or may comprise a machine for the extracorporeal treatment of blood. The system 10 could, for example, alternatively be or comprise a blood processing device or a blood component preparation device or other medical apparatus for fluid delivery and/or collection.

As shown, the exemplary extracorporeal blood treatment system 10 includes computing apparatus 12. The computing apparatus 12 may be configured to receive input from input apparatus 20 and transmit output to display apparatus 22. Further, the computing apparatus 12 may include data storage 14. Data storage 14 may allow for access to processing programs or routines 16 and one or more other types of data 18 that may be employed to carry out exemplary methods and/or processes for use in performing extracorporeal blood treatment, logging historical data, filtering historical data, and displaying historical data. For example, the computing apparatus 12 may be configured to log, or record, data such as flow rates and volumes, to allow a user to select and view various sets of the historical data using the input apparatus 20 (e.g., based on input from the user), and to display the user- selected historical data using the display apparatus 22.

The computing apparatus 12 may be operatively coupled to the input apparatus 20 and the display apparatus 22 to, e.g., transmit data to and from each of the input apparatus 20 and the display apparatus 22. For example, the computing apparatus 12 may be electrically coupled to each of the input apparatus 20 and the display apparatus 22 using, e.g., analogue electrical connections, digital electrical connections, wireless connections, bus-based connections, etc. As described further herein, a user may provide input to the input apparatus 20 to manipulate, or modify, one or more graphical depictions (e.g., windows, regions, areas, buttons, icons, etc.) displayed on the display apparatus 22 to select and/or display historical data. Further, various devices and apparatus may be operatively coupled to the computing apparatus 12 to be used within the computing apparatus 12 to perform one or more extracorporeal procedures/treatments as well as the functionality, methods, and/or logic described herein. As shown, the system 10 may include input apparatus 20 and display apparatus 22. The input apparatus 20 may include any apparatus capable of providing input to the computing apparatus 12 to perform the functionality, methods, and/or logic described herein. For example, the input apparatus 20 may include a touchscreen (e.g., capacitive touchscreen, a resistive touchscreen, a multi-touch touchscreen, etc.), a mouse, a keyboard, a trackball, etc. The input apparatus 20 may allow a user to select and filter various historical data to be viewed on the display apparatus 22 (e.g., displaying a graphical user interface depicting historical data).

Likewise, the display apparatus 22 may include any apparatus capable of displaying information to a user, such as a graphical user interface, etc., to perform the functionality, methods, and/or logic described herein. For example, the display apparatus 22 may include a liquid crystal display, an organic light-emitting diode screen, a touchscreen, a cathode ray tube display, etc. In at least one embodiment, touchscreen apparatus may be overlaid on a display screen allowing a user to touch graphical buttons and icons on the display screen to enable specific actions to occur.

As described further herein, the display apparatus 22 may be configured to display a graphical user interface that includes one or more regions and/or areas used to select and display live and/or historical data for an extracorporeal blood treatment. For example, the graphical user interface displayed by the display apparatus 22 may include, or display, a two-dimensional graph, datasets plotted on the two-dimensional graph, one or more graphical elements, or icons, representing events proximate the two-dimensional graph, a time interval selection region, an event type selection region, an event list region or view, event information areas, a historical data region, an event display region, etc.

Each graph, region, view, button, icon, panel, area, dialog, etc. may be used by a user to select and view historical data on the graphical user interface of the display apparatus 22. As used herein, a "region" of a graphical user interface may be defined as a portion of the graphical user interface within which information may be displayed or functionality may be performed. Regions may exist within other regions, may be displayed separately or simultaneously, etc. For example, smaller regions may be located within larger regions, regions may be located side-by-side, etc. Additionally, as used herein, an "area" of a graphical user interface may be defined as a portion of the graphical user interface located with a region that is smaller than the region it is located within.

The processing programs or routines 16 may include programs or routines for performing computational mathematics, matrix mathematics, standardization algorithms, comparison algorithms, or any other processing required to implement one or more exemplary methods and/or processes described herein. Data 18 may include, for example, historical data, user accounts, licensing information, treatment profiles, bitmaps, videos, calibration data, system configuration information, solutions data, engineering logs, event and alarm data, system pressures, system voltages, system currents, self -test sequence data, user interaction data, treatment state data, monitor usage data, utilization data, software executables, patient information, treatment summary info, treatment run time data, graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein, or any other data that may be necessary for carrying out the one and/or more processes or methods described herein.

In one or more embodiments, the system 10 may be implemented using one or more computer programs executed on programmable computers, such as computers that include, for example, processing capabilities, data storage (e.g., volatile or non-volatile memory and/or storage elements), input devices, and output devices. Program code and/or logic described herein may be applied to input data to perform functionality described herein and generate desired output information. The output information may be applied as input to one or more other devices and/or methods as described herein or as would be applied in a known fashion.

The program used to implement the methods and/or processes described herein may be provided using any programmable language, e.g., a high level procedural and/or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, at least in one embodiment, the system 10 may be implemented using a computer readable storage medium, configured with a computer program, where the storage medium so configured causes the computer to operate in a specific and predefined manner to perform functions described herein. Further, in at least one embodiment, the system 10 may be described as being implemented by logic (e.g., object code) encoded in one or more non-transitory media that includes code for execution and when executed by a processor operable to perform operations such as the methods, processes, and/or functionality described herein.

Likewise, the system 10 may be configured at a remote site (e.g., an application server) that allows access by one or more users via a remote computer apparatus (e.g., via a web browser), and allows a user to employ the functionality according to the present disclosure (e.g., user accesses a graphical user interface associated with one or more programs to process data).

The computing apparatus 12 may be, for example, any fixed or mobile computer system (e.g., a controller, a microcontroller, a personal computer, mini computer, etc.). The exact configuration of the computing apparatus 12 is not limiting, and essentially any device capable of providing suitable computing capabilities and control capabilities (e.g., graphics processing, control of extracorporeal blood treatment apparatus, etc.) may be used.

As described herein, a digital file may be any medium (e.g., volatile or nonvolatile memory, a CD-ROM, a punch card, magnetic recordable tape, etc.) containing digital bits (e.g., encoded in binary, trinary, etc.) that may be readable and/or writeable by computing apparatus 12 described herein.

Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user.

In view of the above, it will be readily apparent that the functionality as described in one or more embodiments according to the present disclosure may be implemented in any manner as would be known to one skilled in the art. As such, the computer language, the computer system, or any other software/hardware which is to be used to implement the processes described herein shall not be limiting on the scope of the systems, processes or programs (e.g., the functionality provided by such systems, processes or programs) described herein.

One will recognize that a graphical user interface may be used in conjunction with the embodiments described herein. The user interface may provide various features allowing for user input thereto, change of input, importation or exportation of files, or any other features that may be generally suitable for use with the processes described herein. For example, the user interface may allow users to select and filter various historical data to be displayed on the display apparatus.

The methods and/or logic described in this disclosure, including those attributed to the systems, or various constituent components, may be implemented, at least in part, in hardware, software, firmware, or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, or other devices. The term "processor" or "processing circuitry" may generally refer to any of the foregoing logic circuitry, alone or in combination with other logic circuitry, or any other equivalent circuitry.

Such hardware, software, and/or firmware may be implemented within the same device or within separate devices to support the various operations and functions described in this disclosure. In addition, any of the described components may be implemented together or separately as discrete but interoperable logic devices. Depiction of different features, e.g., using block diagrams, etc., is intended to highlight different functional aspects and does not necessarily imply that such features must be realized by separate hardware or software components. Rather, functionality may be performed by separate hardware or software components, or integrated within common or separate hardware or software components. When implemented in software, the functionality ascribed to the systems, devices and methods described in this disclosure may be embodied as instructions and/or logic on a computer-readable medium such as RAM, ROM, NVRAM, EEPROM, FLASH memory, magnetic data storage media, optical data storage media, or the like. The instructions and/or logic may be executed by one or more processors to support one or more aspects of the functionality described in this disclosure.

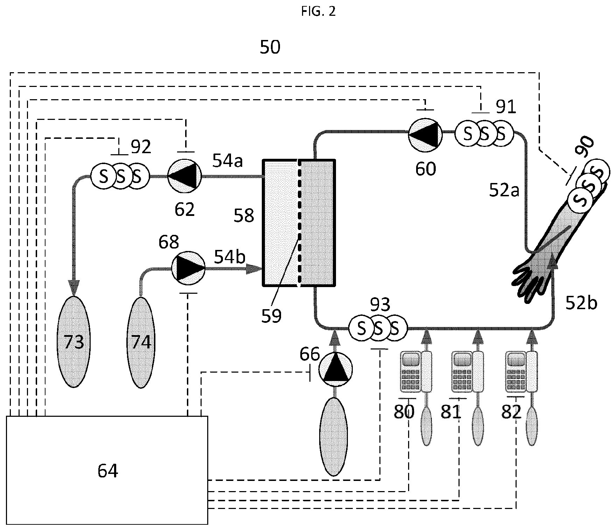

The exemplary systems, and exemplary methods performed, or used, by such exemplary systems, described herein for selecting and viewing of historical data in extracorporeal blood treatment may be generally referred to as dialysis systems. The general term dialysis as used herein includes hemodialysis, hemofiltration, and hemodiafiltration. In dialysis generally, blood is taken out of the body and exposed to a treatment device to separate substances therefrom and/or to add substances thereto, and the blood is then returned to the body. Accordingly, extracorporeal blood treatment systems capable of performing general dialysis are described herein with reference to the exemplary extracorporeal blood treatment system of FIGS. 2-4. Other systems may benefit from the systems, methods, and apparatus described herein and the present disclosure is not limited to any particular fluid processing system.

In the schematic view of FIG. 2, the exemplary extracorporeal blood treatment system 50 generally includes a blood tubing circuit 52 which includes an arterial line 52a and a venous line 52b which are connectable to a patient's vascular system. The apparatus comprises a filtration unit 58 having a primary chamber (blood chamber) and a secondary chamber (dialysate chamber) separated by a semi-permeable filtration membrane 59. The inlet of the primary chamber is connected to the blood withdrawal line or arterial line 52a. Likewise, the outlet of the primary chamber is connected to the blood return line or venous line 52b.

The inlet of the secondary chamber is connected to a fresh dialysate fluid supply line 54b, which in turn is connected to a source 74 for providing a fresh dialysis solution. The outlet of the secondary chamber of the filtration unit 58 is connected to a dialysis solution or spent dialysate line 54a, which conveys a spent dialysis solution to an effluent connector 73.

In the present invention, the filtration unit 58 comprises a membrane 59 which differs from conventional hemodialysis filtration membranes in that it is configured to remove relatively small compounds from blood in the blood tubing circuit 112. The membrane is configured to have a molecular weight cut off (MWCO) of 50 kDa or less or even 40 kDa or less (such as 30 kDa or less, 10 kDa or less, 5 kDa or less or 2 kDa or less).

Flow of blood through the blood line 52a, 52b and filtration unit 58 is governed by a blood pump 60 positioned in the arterial line 52a. Likewise, flow of fresh dialysate fluid from the source 74 through the filtration unit 58 to the effluent collector 73 by a dialysate fluid pump 68 in the supply line 54b, while pressure in dialysate fluid line 54 is governed by a pressure release valve in the spent dialysate line 54a. Alternatively, a second pump 62 could be included in the spent dialysate line 54a instead of a pressure release valve.

There could also be one or more infusion lines 66, 80, 81, 82 that are connected to the venous portion of the blood line 52b. In some embodiments, one or more of these infusion lines are adapted to be directly connected to the vascular system of a patient (not shown in the figures). Each of the one or more infusion lines 66, 80, 81, 82 may comprise separate pumps. In one embodiment, an infusion liquid containing ketone bodies or ketone body derivatives could be infused by such an infusion line.

A system computing unit 64 is provided in communication with the blood pump 60, the dialysate fluid pump 68, and the one or more pumps of the one or more infusion lines 66, 80, 81, 82 and the pressure release valve or additional dialysate pump 68 so as to provide control over those devices in use. In an alternative embodiment, the system computing unit 64 provides the user with suitable settings data via a graphical user interface for the pumps in the one or more infusion lines 66, 80, 81, 82. The system computing unit is also in communication with an input means for the provision of information and instruction to the system processing unit 64. The input means may comprise a graphical user interface such as one which may be controlled by the use of a touch screen arrangement. In some embodiments, the input means may comprise a keyboard. In some embodiments, the input means may include one or more sensors for detecting the concentration of various substances or conditions in the patient's blood and/or in the spent dialysate.

For example, sensors may be provided to detect the concentration of one or more of amino acids, such as glutamine, serine, glycine, arginine glucose, ketones and cytokines in the subject's blood. In particular, such sensors should be adapted for detecting the concentration of one or more of glutamine, glucose and ketone bodies in the subject's blood. Such sensors S, 91, 93 may be provided in the blood line 52 or by the use of separate devices which may be placed elsewhere on the patient's body S, 90. The sensors, to the extent that they are deployed, provide an indication to the system processing unit 64 (either directly or via an additional input means) of the actual blood concentration of the intended analyte. This provision may be made periodically or substantially continuously during treatment.

Suitable sensors and assays for analysing glutamine, glucose and ketone bodies are known in the art. For example, glutamine sensors are disclosed in US, A, 4780191. Examples of glutamine assays are disclosed in US 9995750 and US2016/168619. Examples of sensors and assays for determining concentrations of ketone bodies are disclosed in US 8532731 B2, WO2016/178823, US5326697 and US5618686. Examples of sensors and assays for glucose are disclosed in US2019/328288, US2018/128767 and US2011/105871.

Furthermore, the patient's blood concentration of one or more of amino acids such as glutamine, serine, glycine, and arginine, as well as glucose, ketones and cytokines (preferably at least that of glucose and glutamine) may be measured by withdrawal and separate analysis (e.g. laboratory analysis) of the patient's blood. This method may be especially relevant during long lasting treatments, though depending on the proximity and speed of analysis facilities may be deployed during any treatment.

In another embodiment, one or more sensors S, 92 may optionally be included in the effluent line 54b. This sensor may detect the concentration of one or more of glucose, amino acids such as glutamine, serine, glycine and arginine, ketones or cytokines, as required. The concentration of the relevant analyte measured in the effluent line can be used to determine a representative concentration of the relevant analyte in the patient's blood, such as in the manner described in European patent EP2377563.

When cells in the body are exposed to higher than normal temperatures, changes take place inside the cells. Treatment can be local (just the tumor), regional (as a limb) or whole-body hyperthermia, depending on the extent of the area being treated. Very high temperatures can result in cancer cells going into necrosis/apoptosis (thermal ablation), but high temperatures may also lead to injuries or induce apoptosis/necrosisof normal cells. Therefore, hyperthermia must be carefully controlled.

Hyperthermia is a promising way to improve cancer treatment and today it requires special equipment and is therefore cumbersome to perform. Many clinical trials are ongoing on hyperthermia to treating cancer. An extra corporeal circuit as suggested is well suited to both induce hypothermia in the patient and to control it. To increase blood temperature simultaneous with treating the extracorporeal blood content makes a good combination treatment that can boost the effect from the changed blood content.

With a blood flow of 300 ml/min and a temperature of 43 degrees in the returning blood roughly 120 watt of effect is transferred to the patient and will rise the body temperature readily. The blood temperature could be increased to 45°C for a short amount of time without substantially damaging the blood.

Cooling the patient may be needed to end the treatment but also to modulate a too effective treatment. Cooling will lead to reduction of the metabolic rate in normal cells and cancer cells but subsequently also their oxygen consumption. The mentioned changes will lead to protection of normal healthy cells but will lead to weakness of cancer cells against e.g. conventional cancer treatments but also Cancer Dialysis and reduction of glucose and glutamine and oxygen. Cooling could also lead to less adverse side-effects to the conventional cancer treatment given to the patient but also withstand more aggressive treatments of Cancer Dialysis and conventional cancer treatment. The present drawings do not indicate any means for cooling or heating blood but such means are well-known to the skilled person and are frequently used in connection with some types of extracorporeal blood treatment, such as continuous renal replacement therapy (CRRT).

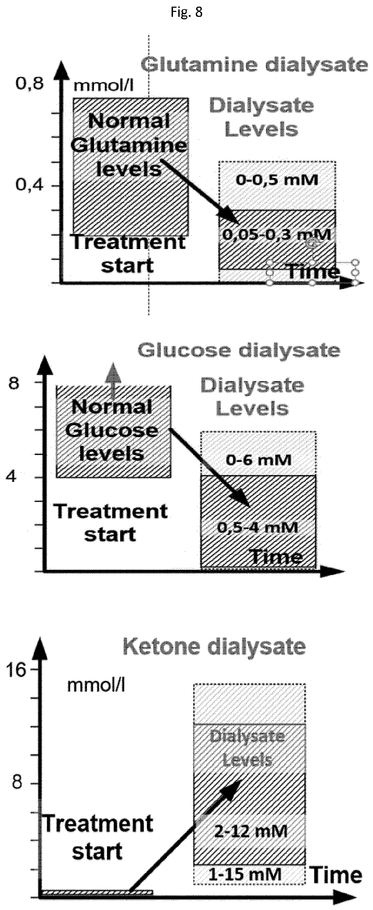

FIGS. 8A, 8B and 8C illustrate the therapy goals and principles of the present invention. Each diagram discloses examples of normal conditions regarding concentration of a specific blood component. FIG. 8A shows that the patient at the onset of the treatment typically has an actual blood concentration of glutamine GLNₐ within the range of 0.20 - 0.8 mM. During treatment, the actual blood concentration of glutamine is reduced to a desired value GLN_{b}, which is within the range of 0.1 - 0.5 mM, and for example within the range of 0.15 - 0.3 mM. FIG. 8B shows that the patient at the onset of the treatment typically has an actual blood concentration of glucose GLUCOSEₐ within the range of 4 - 8 mmol/l. During treatment, the actual blood concentration of glucose is reduced to a desired value GLUCOSE_{b}, which typically is within the range of 2 - 4 mmol/l. Finally, FIG. 8C shows that the blood initially hardly contains any ketone body such as beta-hydroxy-butyric acid or physiologically acceptable salt or ester thereof, such as the sodium salt. Accordingly, the actual value of concentration of ketone bodies in the patient's blood KETONEₐ is about 0. Typically, the fresh dialysate does not contain any ketone bodies or only small amounts. During treatment, the blood concentration of such ketones may rise to a desired value KETONE_{b} within the range of 1 - 15 mmol/I such as within the range of 2 - 12 mM, by infusion of a solution of ketone bodies and/or ketone body derivatives.

In one embodiment, the actual blood concentration of glutamine, GLNₐ, and/or the actual blood concentration of glucose, GLUCOSEₐ, could fall below the desired values GLN_{b}, and/or GLUCOSE_{b}. Then, these actual blood concentration values GLNₐ and/or GLUCOSEₐ could be increased to desired values GLN_{b} and/or GLUCOSE_{b} by infusion of solutions containing glucose and/or glutamine or pharmaceutically acceptable glutamine-containing compounds.

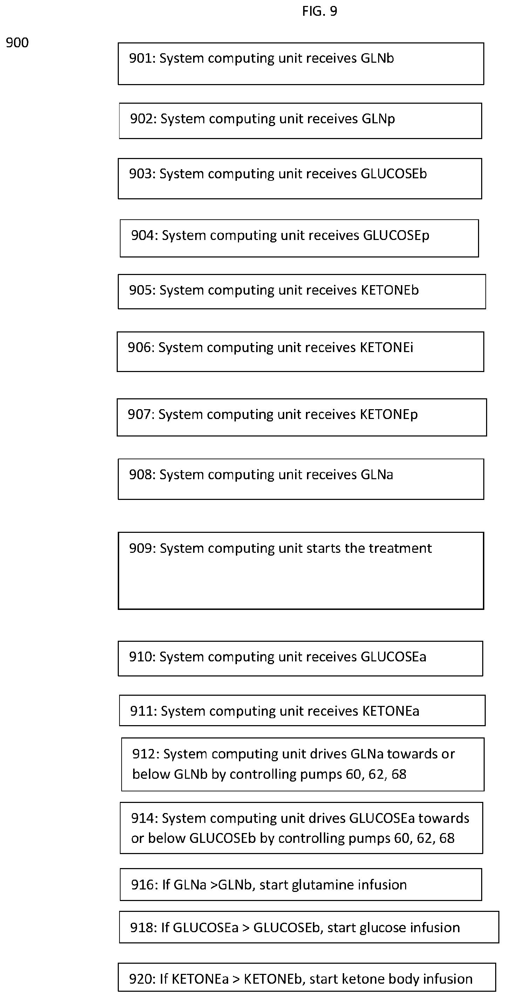

FIG. 9 discloses the flow chart of an example of an algorithm 900 of a control process in accordance with the present invention Before starting the treatment, the system computing unit 64 is adapted for receiving treatment goal concentrations or desired blood concentrations of the key compounds. Typically, these treatment goal concentrations are entered using the user interface.

Hence, in step 901, the system computing unit 64 receives a desired blood concentration value of glutamine, GLN_{b}.

In step 903, the system computing unit 64 receives a desired blood concentration value of glucose, GLUCOSE_{b}.

In step 905, the system computing unit 64 receives a desired blood concentration value of ketone bodies, KETONE_{b}.

Before starting the treatment, the system computing unit 64 is also adapted for receiving concentration values in the fresh dialysate liquid for the above key components. Typically, these concentration values are also entered using the user interface.

Hence, in step 902, the system computing unit 64 receives the concentration value of glutamine, GLNₚ, in the fresh dialysate liquid.

In step 904, the system computing unit 64 receives the concentration value of glucose, GLUCOSEₚ, in the fresh dialysate liquid.

In step 907, the system computing unit (64) is adapted for receiving a concentration value KETONEₚ,,representing the concentration of a ketone body, such as acetoacetate, beta-hydroxybutyrate or pharmaceutically acceptable derivatives, esters and salts thereof in fresh dialysate liquid;
In step 906, the system computing unit 64 receives the concentration value of ketone bodies, KETONEᵢ, in an infusion liquid to be infused in the extracorporeal blood line (52b) or directly into the vascular system of the subject/patient to be treated.

Before starting the treatment, as well as optionally during the treatment, the system computing unit 64 is adapted for receiving actual concentration values determined from a patient's blood. These concentration values could be obtained after taking blood samples followed by analysis in separate analysis units. The concentration values are then entered manually through the user interface. In some embodiments, the system computing unit is connected to one or more such separate analysis units and may therefore receive data directly therefrom. In some embodiments, the blood circuit 52a, 52b, and/or the dialysate circuit 54a, 54b may comprise suitable sensors S, 90, 91, 92, 93, which are connected to system computing unit 64 which receives the data. The system computing unit 64 typically controls the treatment Based on the most recently received actual values GLNₐ, GLUCOSEₐ, and KETONEₐ.

Hence, in step 908, the system computing unit 64 starts the treatment.

In step 909, the system computing unit 64 is adapted for receiving an actual blood concentration value of glutamine, GLNₐ, from a patient.

In step 910, the system computing unit 64 is adapted for receiving an actual blood concentration value of glucose, GLUCOSEₐ, from a patient.

In step 911, the system computing unit 64 is adapted for receiving an actual blood concentration value of ketone bodies, KETONEₐ, from a patient.

In step 912, the system computing unit 64 controls the blood pump 60 and the dialysate liquid pump 62, 68 in such a way that GLNₐ is driven towards or below GLN_{b}.

In simultaneous step 914, the system computing unit 64 controls the blood pump 60 and the dialysate liquid pump 62, 68 in such a way that GLUCOSEₐ is driven towards or below GLUCOSE_{b}.

In simultaneous step 916, the system computing unit 64 monitors GLNₐ, and if GLNₐ is lower than GLN_{b}, the system computing unit initiates and maintains infusion of a composition containing glutamine or glutamine-containing compounds into the extracorporeal blood circuit 52a, 52b.

In simultaneous step 918, the system computing unit 64 monitors GLUCOSEₐ, and if GLUCOSEₐ is lower than GLUCOSE_{b}, the system computing unit initiates and maintains infusion of a composition containing glucose into the extracorporeal blood circuit 52a, 52b.

In simultaneous step 920, the system computing unit 64 monitors Kₐ, and if Kₐ is lower than K_{b}, the system computing unit initiates and maintains infusion of a composition containing ketone bodies into the extracorporeal blood circuit 52a, 52b or direct into the vascular system of the subject to be treated.

In some embodiments, the concentration of other substances in the blood may be controlled. For example, it has been found to be beneficial to reduce from normal the concentration of amino acids such as serine, glycine and arginine

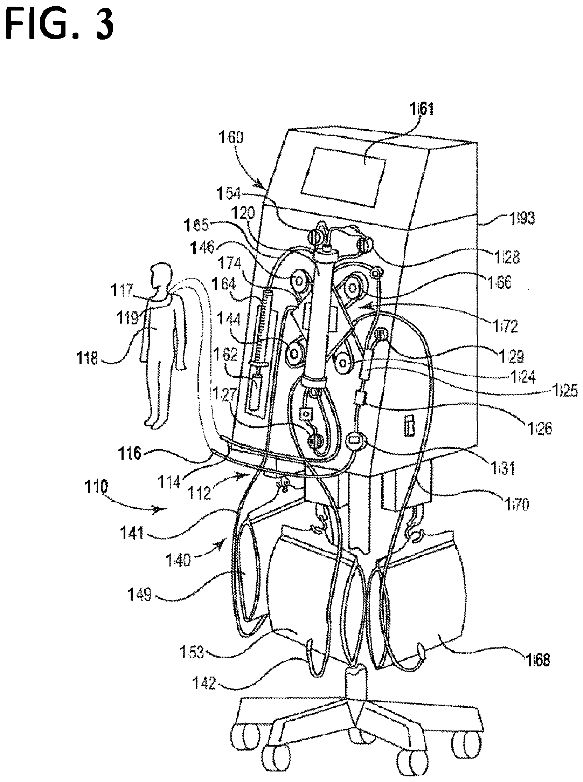

In the perspective and partial front views of FIGS. 3-4, an exemplary extracorporeal blood treatment system 110 that may implement the treatments and graphical user interfaces as described herein generally includes a blood tubing circuit 112 having first and second tubing segments 114 and 116 which are both connected to the vascular system of a patient 118 via access and return devices 117 and 119, respectively. Devices 117 and 119 may be cannulas, catheters, winged needles or the like as would be understood by one skilled in the art. Tubing segments 114 and 116 are also connected to a filtration or processing unit 120. In dialysis, filtration unit 120 is a dialyzer, which is also often referred to as a filter.

Numerous other component devices of blood circuit 112 are also included as, for example, pressure sensors 127, 128, 154, 129. Further sensors may be provided to monitor the concentration of glucose and/or glutamine; ketones (such as hydroxybutyrate (BHB) acetoacetate and acetone) and/or cytokynes (such as tumour necrosis factors (TNF) (e.g. TNF-α), interleukins (for example IL-6)). As noted above, however, the sensors may be placed in direct contact with the patient rather than in the blood circuit 112. Where they are present, the sensors are operatively connected to the computing apparatus 12 by wireless connection or a physical connection.

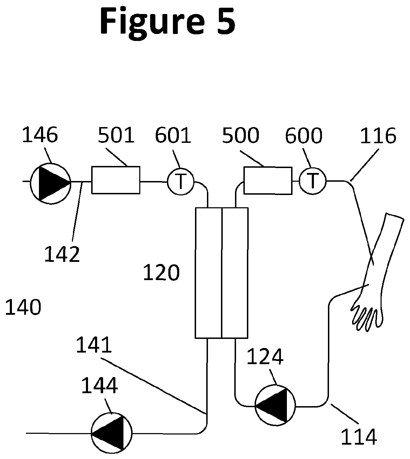

As is shown in schematic form in FIG. 5, the blood circuit 112 comprising blood access line 114 and blood return line 116 may also include a thermal management system 500 for heating or cooling blood within the blood circuit 112. The thermal management system 500 is operatively connected to the computing apparatus 12, 64. Such a thermal management system may be used to heat blood returning to the patient, such as to around 37°C. Such a system could also be used to cool with returning blood down to 20 °C or to increase the returning blood temperature to 43 °C, thereby warming the patient to a temperature up to 38 - 40°C. Furthermore, the dialysate circuit may also include a second thermal management system 501 in order to ensure that the blood has achieved the desired temperature after passing the blood filter 120.

Also shown in FIGS. 3-4 is the dialysate fluid or filtrate side of system 110 which generally includes a dialysate fluid circuit 140 having first and second dialysate fluid tubing segments 141 and 142. Each of these tubing segments is connected to the filtration unit 120 on the opposite side of the membrane to the blood circuit 112 segments 114, 116. In these FIGS. 3-4, a respective fluid pump 144, 146 is operatively associated with each of these tubing segments 141 and 142.

First tubing segment 141 is also connected to a dialysate fluid source (e.g., fluid bag 149), which may include electrolytes or other treatment compounds pre-mixed therein. Second tubing segment 142 is connected to a waste collection device (e.g., a waste container such as a bag 153). A pressure sensor 154 may also be disposed in second dialysis fluid tubing segment 142.

FIGS. 3-4 show a system that is common as a basic model for numerous dialysis procedures. Additional fluid lines, circuits, and components may be added (or deleted) to increase therapy options. In particular, in the present invention an additional line may be provided for the supply to the patient (such as by addition to the blood circuit 112) of ketones (for example hydroxybutyrate (BOHB), acetoacetate and/or acetone). The supply of ketones to the patient is important because it reduces the requirement for the patient to be in a condition of dietary induced ketosis before the start of the treatment. In embodiments where no separate supply of ketones is provided, the fluid source bag 149 may also include one or more ketones (such as sodium BOHB) in solution for diffusion through the membrane into the blood circuit.

One or more of the dialysate fluid source 149, waste container 153 and replacement fluid container 168 may be provided on scales 400 which are in operative communication with the computing apparatus 12. Any additional source of ketones may also be provided on such scales 400. This allows the mass of the fluid in each container to be monitored in use to provide an accurate record of the quantity of each fluid used or collected.

Further, as shown in FIGS. 3-4, the system 110 includes an extracorporeal blood control apparatus 160 that provides numerous treatment options, which may be controlled and/or monitored via the control/display screen 161 (e.g., a control apparatus or controller provided in a system housing 193). Touch-screen controls may be incorporated herewith and/or other conventional knobs or buttons (not shown) may be used (e.g., graphical user interfaces may be displayed via a touchscreen as described herein). Other and more detailed information regarding an example apparatus 160 may be found in U.S. Pat. No. 5,679,245; U.S. Pat. No. 5,762,805; U.S. Pat. No. 5,776,345; and U.S. Pat. No. 5,910,252; inter alia.

A general dialysis treatment procedure as performed, for example, with an apparatus described with reference to FIGS. 3-4 will be generally described for exemplary purposes. First, blood is removed from the patient 118 via access device 117 by, e.g., the blood pump 124, and flows through access line 114 to the filter 120. Filter 120 processes this blood according to a selected one or more of a number of extracorporeal blood treatment profiles (e.g., selected and controlled via screen interface 161 of control apparatus 160).

The treatment profiles also involve the reduction of glutamine and glucose in the patient's blood. The concentration of glutamine is reduced to a value within the range of 0.1 - 0.5 mM and preferably to a value within the range of 0.15 - 0.3 mM. The blood concentration of glucose is reduced to a concentration within the range of 1 - 6 mM, and preferably within the range of 2 - 4 mM. This treatment has a much more pronounced and adverse effect on cancer cells than healthy cells in the body, because glycolysis and glutaminolysis pathways are enhanced in many cancer cells. While glycolysis is enhanced to satisfy the increasing energy demand of cancer cells, glutaminolysis is also enhanced in many cancer cells and can provide biosynthetic precursors but also an important role in maintaining Reactive Oxygen Species (ROS) haemostasis. To force cancer cells into apoptosis by increasing ROS production in cancer cells is an important target for both radio and chemotherapies. Lastly glutamine may act as a source of ATP during low levels of glucose. In some embodiments, the treatment profiles ensure that the patient's nutritional needs can be met through the provision of a suitable parenteral nutrition composition to the blood line or as a separate infusion directly into the vascular system of a patient. In some embodiments, a blood concentration of ketone bodies is maintained at a value within the range of 1 - 15 mM, and preferably within the range of 2 - 12 mM during treatment. While a patient would be expected to produce its own ketone bodies (e.g. from consumed or infused lipids or stores of body fat) when exposed to such low blood glucose concentrations, the biological process to produce ketone bodies in sufficient amount can take time to commence. The introduction of ketone bodies during the treatments described herein ensures that the patient timely has a sufficient source of energy for continued vital function even where the blood glutamine and blood glucose concentrations are reduced at an unnaturally high speed.

In certain treatments (especially those in which there is a high blood ketone body concentration maintained) a supply of one or more pharmacological agents is provided to the patient to aid in the reduction of blood glucose concentration. Such glucose reducing agents preferably include biguanides, alpha-glucosidase inhibitors, SGLT2 inhibitors and dopamine agonists. Such agents may be supplied by the system by inclusion in the dialysate fluid source 149, the additional source of ketones (if present) or as an additional infusion to be supplied to the blood circuit 112 or otherwise to the patient.

An example biguanide is metformin. Example alpha-glucosidase inhibitors include acarbose or miglitol. Example SGLT2 inhibitors include canagliflozin, dapagliflozin and empagliflozin. Example dopamine agonists include bromocriptine.

In some preferred treatments anti-glycolytic agents may be provided to the patient to further inhibit glycolytic activity in tumour cells. Such agents may be supplied by the system by inclusion in the dialysate fluid source 149, the additional source of ketones (if present) or as an additional infusion to be supplied to the blood circuit 112 or otherwise to the patient.

Following the treatment, the system returns the processed or treated blood to the patient 118 through return line 116 and return device 119 inserted in or otherwise connected to the vascular system of the patient 118.

The blood flow path to and from the patient 118, which includes the access device 117, the access line 114, the blood pump 124, the filter 120, as well as the return line 116 and return device 119 back to the patient, forms the blood flow circuit 112. Pressure sensors may be used to sense various pressures in the system 110. For example, the pressure sensor 127 may be connected in the access line 114 and allow the fluid pressure in the access line 114 to be monitored and the second pressure sensor 128 may be connected in the blood circuit 112 between the first blood pump 124 and the blood entrance into the filter 120 and may be used to detect and monitor the pressure of the blood supplied to the entrance of the filter 120.

The system 110 may further include a deaeration chamber 125 in the return line to provide a conveyance path that operates like a vortex to propel air out of the blood. Post-filter replacement solution may be added into the deaeration chamber on the top of the blood to prevent an air/blood interface. A deaeration chamber monitor line 191 may connect the deaeration chamber 125 to an internal pressure transducer within the system housing 193 using a connection apparatus, such as, for example, a return pressure port 129. This enables return pressure monitoring, and removal of air from the deaeration chamber, if needed. A return clamp 131 connected in the blood circuit 112 selectively allows or terminates the flow of blood through the blood circuit 112 (e.g., return clamp 131 may be activated whenever air is detected in the blood by bubble detector 126).

Further, a pump 162 may be connected to an anticoagulant container 164 to deliver anticoagulant through an anticoagulant line 165 to the blood in tubing segment 114 and a pump 166 may deliver replacement fluid from a replacement fluid container or bag 168 through a replacement fluid line 170. The secondary flow circuit 140 is also shown in FIGS. 3-4 as it interacts with filter 120. The secondary flow circuit 140 is connected to the secondary chamber of filter 120. Matter extracorporeally removed from the blood is removed from the secondary chamber of filter 120 through the outlet tubing segment 142 of the secondary flow circuit 140, and matter extracorporeally added to the blood is moved into filter 120 through inlet tubing segment 141 of the secondary flow circuit 140.

The secondary flow circuit 140 generally includes the fluid source such as bag 149, inlet fluid line 141, third pump 144, the secondary chamber of the filter 120, a waste fluid line 142, pressure sensor 154, fourth pump 146, and the waste collection device such as container 153. The source fluid bag 149 may contain a sterile dialysate fluid, generally isotonic to blood, into which blood impurities will diffuse through the semi-permeable membrane of the filtration unit 120. The fluid source bag may also include one or more ketones in solution for diffusion through the membrane into the blood circuit.

The pump 144 is connected in inlet fluid line 141 for delivering dialysate fluid from the dialysate fluid source 149 into an entrance to the filter 120. The waste collection container 153 is provided to collect or receive matter from the blood transferred across the semi-permeable membrane in filter 120 and/or to receive the used dialysate fluid after it has passed through the filter 120. The fourth pump 146 is connected to the waste collection line 142 for moving spent dialysate from the filter 120 into the waste collection container 153. The pressure sensor 154 may also be located in the waste collection line 142 for the purpose of monitoring the pressure in the secondary chamber of filter 120.

The filtration unit 120, the flow tubing lines, and the other components in the primary and secondary flow circuits 112 and 140 described herein (with the exception, for example, of the pumps and perhaps a few other items) may be formed as an integral, replaceable unit (e.g., an extracorporeal blood set). This integral replacement unit may be referred to herein as a "therapy set." An example of such a therapy set, or integral replaceable unit, is described in greater detail in U.S. Pat. No. 5,441,636 entitled Integrated Blood Treatment Fluid Module (see also, U.S. Pat. No. 5,679,245, entitled Retention Device for Extracorporeal Treatment Apparatus).

Therapy sets for use in performing different therapies may be available depending on the system configuration. As can generally be appreciated from FIGS. 3-4, the integrated tubing and filter module (identified by the reference numeral 172) includes the filter 120 and all the tubing and related components described above which are connectable to apparatus 160. For example, the filter and tubing may be retained on a plastic support member 174 which is, in turn, connectable to apparatus 160 (e.g., connectable to the system housing 193 of the apparatus 160). The therapy sets may also include the sensors monitoring the concentration of glucose and/or glutamine; ketones (such as hydroxybutyrate (BHB) acetoacetate and acetone) and/or cytokines (such as tumour necrosis factors (TNF) (e.g. TNF-α), interleukins (for example IL-6)). Such sensors may be configured to monitor the concentration of the listed components in the blood circuit or may be configured to monitor the concentration of the listed components by direct interfacing with the patient's body.

When in the operative position connected to apparatus 160, flexible fluid conducting tubing lines to and from the filtration unit 120 are held in operative, pump communicative loops for operative contact with the peristaltic pumping members of the pumps 124, 144, 146 and 166 to cause the fluid to flow through the primary (blood) and secondary (dialysate fluid) circuits 112 and 140. Module 172, including filter 120 and all the tubing lines and associated flow components may be disposable after use.

The peristaltic pumping members of pumps 124, 144, 146, and 166 may be fixedly disposed on apparatus 160 (without the disposable tubing loop components) and may be re-usable. In general, electrical, mechanical, or electromechanical components are also fixedly disposed in or on apparatus 160 (e.g., connectable to the system housing 193 of the apparatus 160). Examples of such components include the display screen 161 (e.g., a touchscreen), the bubble detector 126, line clamps 131 and connection apparatus for coupling to pressure sensor apparatus used to implement pressure sensors 127, 128, 129, 154, etc. Couplings may also be provided for any sensors that are required or preferred.

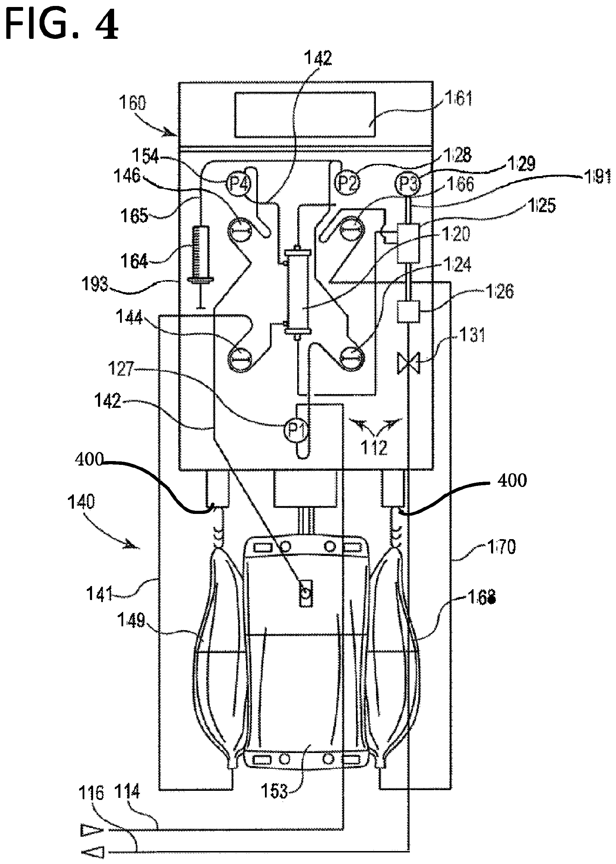

As is noted above, access 117 and return 119 devices may comprise catheters. In some embodiments, the catheters may comprise occlusion catheters, such as balloon occlusion catheters. This arrangement allows for the delivery of the access 117 and return 119 devices to the site of the tumour (for example via entry through the femoral artery or vein) in the manner shown in FIG. 4. This allows for localised reduction in blood glucose concentration around the tumour and thus provides the potential to lower blood glucose and/or glutamine concentration to even lower levels.

### Reference Example 1

A study was made of the sensitivity of different human cancer cell lines to the presence of glucose, glutamine and ketones in the cell culture medium with concomitant depletion of selected nutrients, to mimic the conditions obtained with cancer dialysis.

Study 1 was performed on a selection of human cancer cell lines established from renal cell carcinoma, colon carcinoma and glioblastoma. In the first study the effect on cell viability of growth in the presence of increasing concentration of the ketone β-hydroxybutyrate, with the concomitant restriction of glucose and glutamine levels. The addition of citrate to the cell culture medium was also tested. Cells were cultured under these conditions for three days, thereafter cell viability was determined. In the first study, the main effect on cell viability was found when glutamine was depleted from the culture medium.

### Materials and methods

### Cell culture conditions

Cell lines established from human colon carcinoma (HCT15, NCI-H508 and COLO205), renal cell carcinoma (769-P, 786-O and RCC4) and glioblastoma (LN-18, A-172 and U-118MG) were selected for the analysis. All cell lines were obtained from American Type Culture Collection (ATCC, LGC standards, UK) except for RCC4 and HCT15 that was purchased from Sigma-Aldrich (Merck, Germany). In addition, primary human renal cell carcinoma (RCC) cells isolated from patient nephrectomies were included in the study. The culture conditions were as recommended by American Type Culture Collection (ATCC) were followed, that is to grow cells in DMEM medium with the addition of 1mM sodium pyruvate, which was also added to the RPMI-1640 medium in order to keep the conditions more similar 769-P, RCC4, LN-18, A-172, U-118MG and the primary RCC cells were cultured in DMEM high glucose medium, while 786-O HCT15, NCI-H508 and COLO205 were cultured in RPMI-1640 medium according to the recommendations from ATCC. To both media 1% penicillin-streptomycin and 10% calf serum was added. Cells were expanded and aliquots frozen according to standard procedures.

Optimal seeding density was determined for each cell line in 96 well plates according to the "Protocol for optimizing cell seeding densities to ensure Log-phase growth". This Protocol is as follows:
- Prepare a single-cell suspension and measure cell counts/viability.
- Dilute cells to approx 160,000 cells/mL in complete media. Add 200 µL of cells to the top row of a 96-well plate. Aliquot 100 µL of complete media into all other wells. A small number of media-only control wells are required on each plate to act as a blank.
- Repeatedly, dilute the cell preparation 1 part in 2 down the plate using a 12-well channel pipette, i.e., 100 µL cells added to 100 µL media in the row below. Then, add 50 µL of complete media to all wells. Cover the plate.
- Incubate the plate overnight at 37°C, 5% CO₂.
- Add 50 µL of fresh media to the plate wells to achieve a final volume of 200 µL and incubate for 72 h at 37°C, 5% CO₂.
- Measure viability using Cell Titer Glo assay according to manufacturer's protocol.
- Plot log cell number against luminescence intensity to identify the concentration of cells at which log growth is achieved.

CellTiter-Glo Luminescent cell viability assay (Promega) was used as a readout of viability.

### Study 1.

For each cell line, optimal number of cells as determined above were seeded into 96-well plates on day 0. The following day, cells were washed in PBS and the media was changed to DMEM (Fisher Scientific) or RPMI-1640 medium (Saveen Werner) without glucose or L-glutamine, with the addition of nutrients as outlined in Table 3 and the file "plate overview". Three wells were treated for each condition. After 3d incubation in test condition medium, with daily medium changes, cell viability was determined using the CellTiter-Glo viability test. The experiment was repeated three times for each cell line.

**Table 1. Cancer cell lines and culture medium**

| *kidney* | |
|---|---|
| 769-P | DMEM high glucose |
| 786-O | RPMI-1640 |
| RCC4 | DMEM high glucose |

| *Colon* | |
|---|---|
| HCT15 | RPMI-1640 |
| NCl-H508 | RPMI-1640 |
| COLO205 | RPMI-1640 |

| *Brain* | |
|---|---|
| CRL2610 (LN-18) | DMEM high glucose |
| A-172 | DMEM high glucose |
| U-118MG | DMEM high glucose |

**Table 2. Normal culture medium content of selected nutrients.**

| *Medium* | *Glucose(g*/*L)* | *L-glutamine (mM)* | *NaHCO3 (g*/*L)* | *HEPES (mM)* | *NaPyr (mM)* |
|---|---|---|---|---|---|
| RPMI-1640 | 2 (11mM) | 2 | 2 | 25 | 0(1) |
| DMEM high glucose | 4,5 (25mM) | 4 | 3,7 (1,5) | 0 | 1 |

**Table 3 Test conditions Study 1.**

| | | | BOHB (mM) | |
|---|---|---|---|---|
| | Glucose* | 0 | 4 | 12 |
| w glutamine | 100% | +/-citrate | | |
| | 25% | | +/-citrate | |
| | 0% | | | +/-citrate |
| w/o glutamine | 100% | +/-citrate | | |
| | 25% | | +/-citrate | |
| | 0% | | | +/-citrate |

Matrix showing the combination of different growth conditions used in Study 1. *The amount of glucose is presented as the percentage of the concentration present in the standard culture medium for each cell line. Where indicated, 1mM of citrate was added to the culture medium. The indicated nutrients were added to DMEM (Fisher Scientific) or RPMI-1640 medium (Saveen Werner) without glucose or L-glutamine.

**Table 4 product order information**

| **product** | **company** | **prod no** |
|---|---|---|
| | | |

| *CELL LINES* | | |
|---|---|---|
| 786-O [786-0] (ATCC^{®} CRL-1932^{™}) | ATCC | CRL-1932 |
| COLO 205 (ATCC^{®} CCL-222^{™}) | ATCC | CCL-222 |
| A-172 [A172] (ATCC^{®} CRL-1620^{™}) | ATCC | CRL-1620 |
| 769-P (ATCC^{®} CRL-1933^{™}) | ATCC | CRL-1933 |
| NCI-H508 [H508] (ATCC^{®} CCL-253^{™}) | ATCC | CCL-253 |
| LN-18 (ATCC^{®} CRL-2610^{™}) | ATCC | CRL-2610 |
| U-118 MG (ATCC^{®}HTB-15^{™}) | ATCC | HTB-1 5 |
| RCC4 plus vector alone Renal cell carcin | sigma | 03112702-1VL |
| HCT-15 HUMAN COLON ADENOCARCINOMA | sigma | 91030712-1VL |
| | | |

| *CELL CULTURE MEDIUM* | | |
|---|---|---|
| DMEM/high glucose with 4.0mM L-glut, with Sodium Pyruvate | Nordic Biolabs | SH30243.01 |
| RPMI 1640 with 25 mM HEPES with L-Glut | Nordic Biolabs | SH30255.01 |
| 500ML DMEM WO GLUC & PHENOL RED | Fisher Sc | 12307263 |
| RPMI Medium 1640, w/o D-Glucose, w/o L-Glutamine,500ml | saveen werner | 01-101-1A |
| | | |

| *CELL CULTURE PLASTICS* | | |
|---|---|---|
| 96-well plates white, clear bottom, with lid | Fisher Sc (Corning) | 10517742 |
| Opaque white tape, BackSeal backing tape | perkin elmer | 6005199 |
| cell strainer 40um | Saween Werner | 93040 |
| cell strainer 20um filcons | BD | 340621 |
| | | |

| *VIABILITY ASSAY* | | |
|---|---|---|
| Cell titer Glo | Promega | G7571 |
| | | |

| *CHEMICALS AND ADDITIVES* | | |
|---|---|---|
| DMSO | sigma | D2650-5x5ml |
| Glucose | | |
| L-glutamine | Fisher Sc | 11500626 |
| PEST | Nordic Biolabs | SV30010 |
| trypsin | Nordic Biolabs | SH30042.01 |
| sodium citrate | sigma | PHR1416-1g |
| DL-B-hydroxybutyric acid sodium crystall | sigma | H6501-5g |

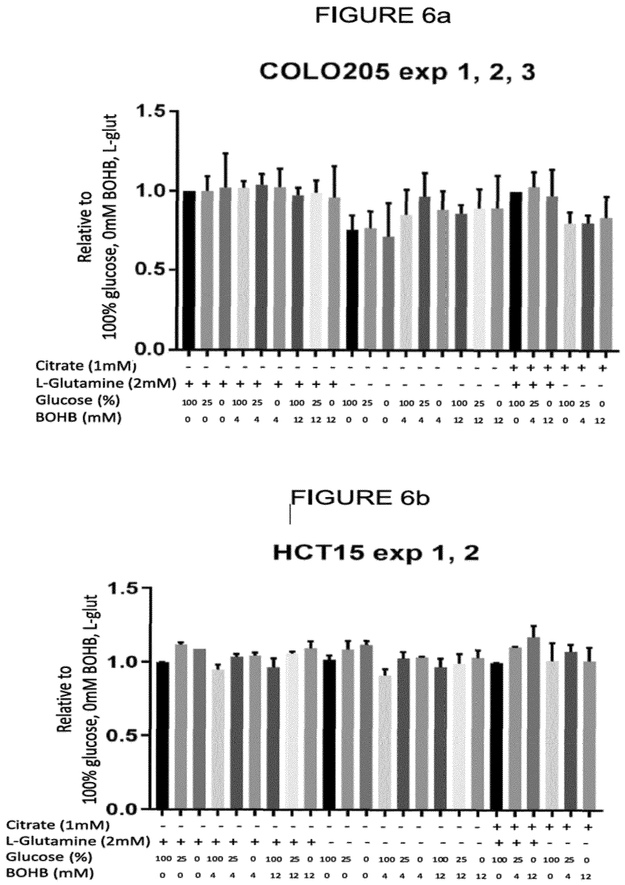

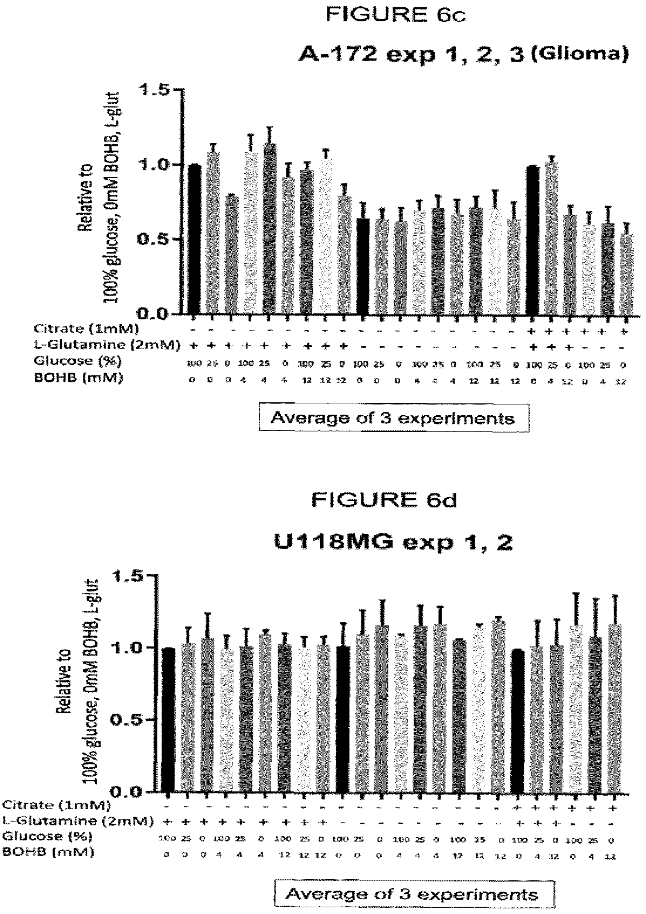

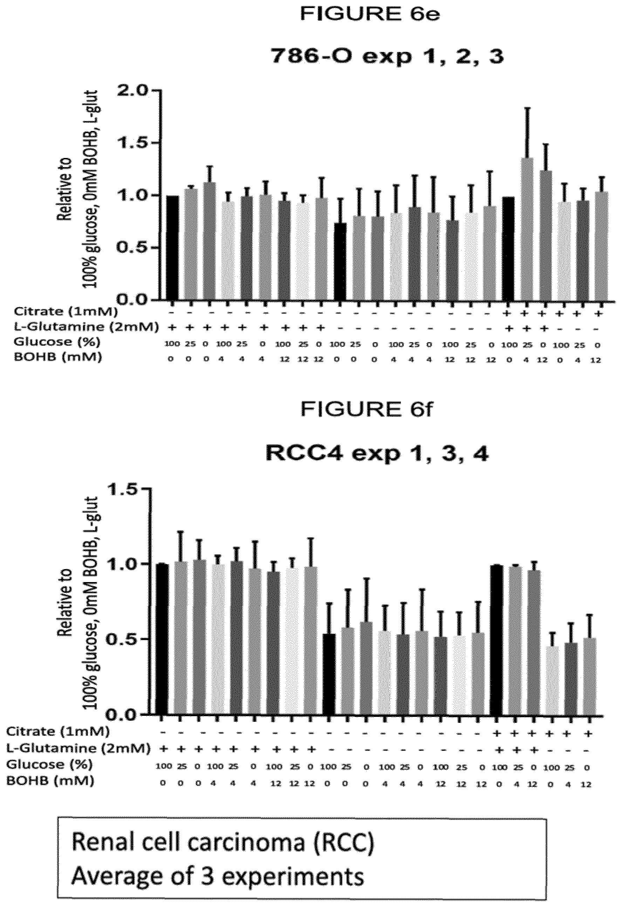

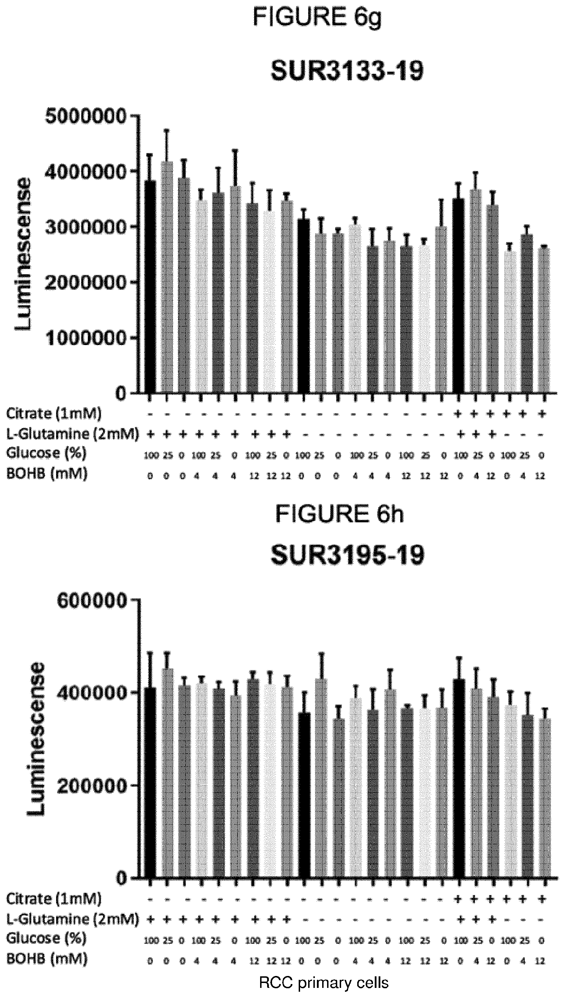

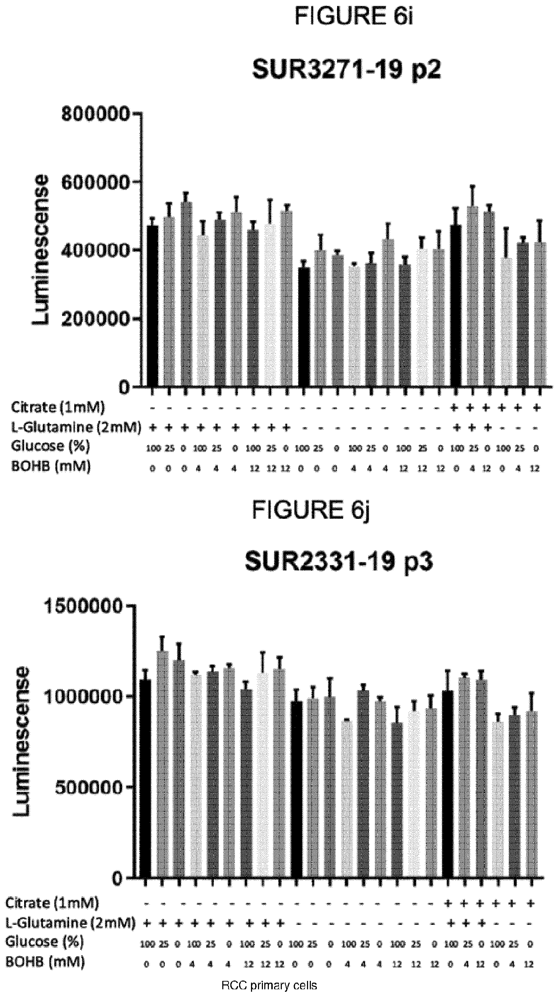

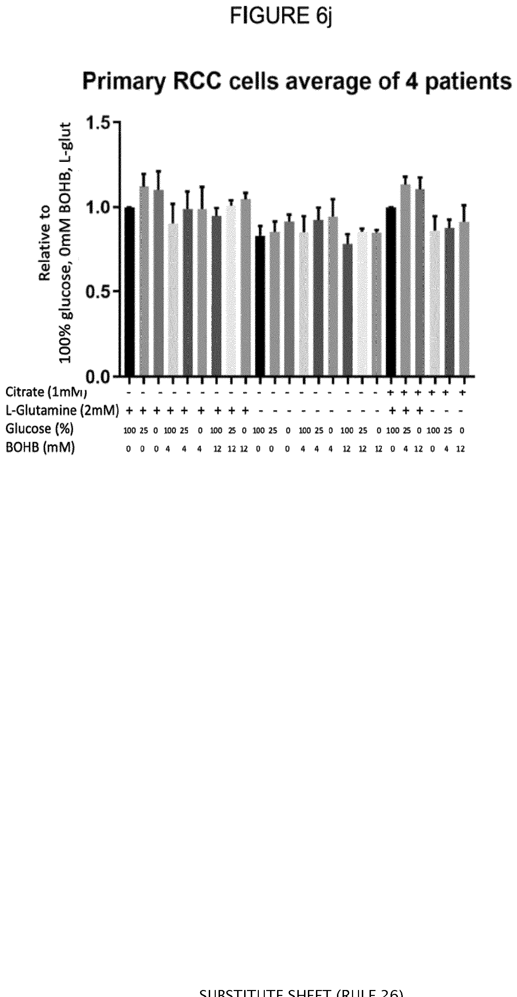

The results of Study 1 are presented in the charts shown in FIG. 6. A clear link is shown between a reduction in glutamine present and the cell culture medium and a reduction in the proliferation of cells.

### Study 2

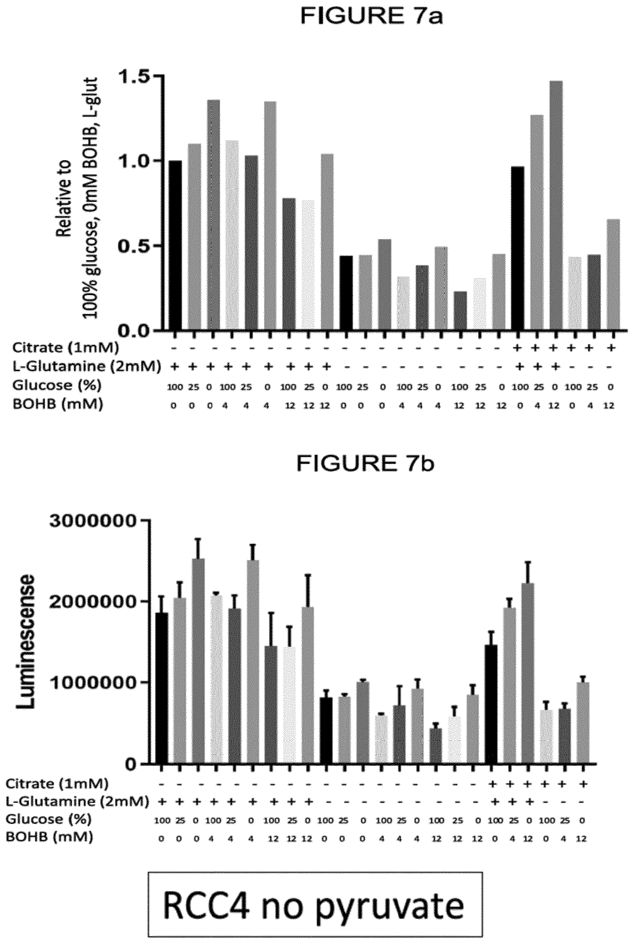

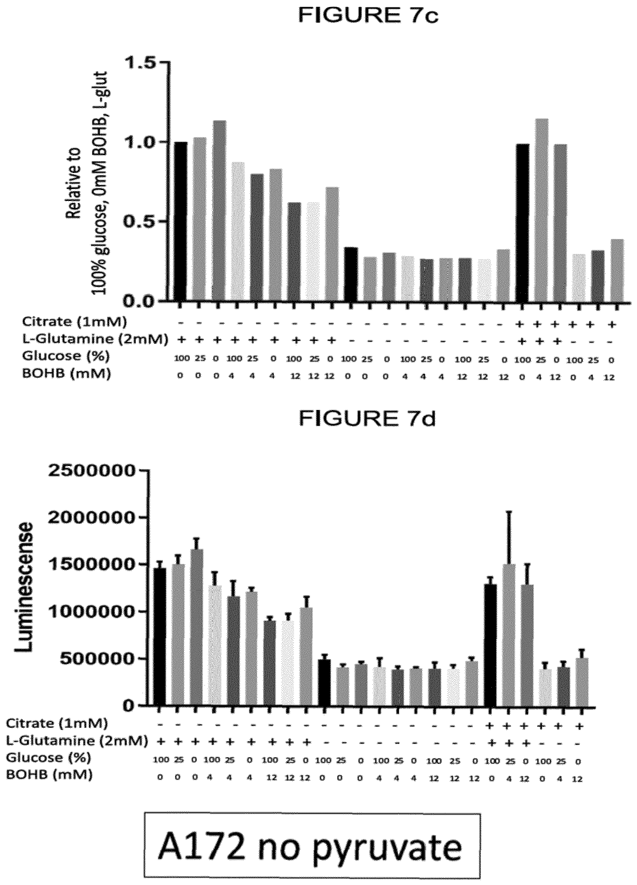

As noted above, in Study 1, the culture conditions recommended from ATCC were followed. However, pyruvate is a potential source of energy that might impact the results. Therefore, in Study 2, the same culture conditions as in Study 1 were tested in two of the cell lines, A172 (glioblastoma) and RCC4 (renal cell carcinoma) in DMEM medium without the addition of sodium pyruvate. The results are presented in FIGS. 7. As in Study 1, a clear link is shown between a reduction in glutamine present and the cell culture medium and a reduction in the proliferation of cells. However, in the absence of pyruvate in the cell culture medium, the results are far more pronounced. It also appears that an increase in the concentration of the BOHB ketone also suppresses the proliferation of cells.

This disclosure has been provided with reference to illustrative embodiments and is not meant to be construed in a limiting sense. As described previously, one skilled in the art will recognize that other various illustrative applications may use the techniques as described herein to take advantage of the beneficial characteristics of the apparatus and methods described herein. Various modifications of the illustrative embodiments, as well as additional embodiments of the disclosure, will be apparent upon reference to this description.

### Example 2

### Study design

### Growth medium

To study the effect of a nutrient restricted ketogenic environment on cancer cell growth *in vitro,* three different cell culture media were formulated. Medium A was full RPMI1640 medium that the cell lines routinely are cultured in. Medium B was used as an approximation of the conditions found in normal human serum. The levels of glucose, glutamine, serine, glycine and arginine were adjusted to match normal physiological levels found in human serum. These nutrients were selected based on their reported use as energy source and effects on cancer cell metabolic state. Medium C was used to emulate the ketogenic nutrient restricted Cancer Dialysis condition. Here, the levels of the selected nutrients were reduced to half of the physiological levels in Medium B, and the ketone body BOHB was added.

The composition of each medium is described in Materials and Methods and listed in Table 5-6.

### Oxygen levels

Human cancer cell lines are routinely established and cultured at atmospheric oxygen levels (21% O₂), yet, the physiological oxygen levels in tissue is considerably lower and varies from 3-13% [Ward, Biochim Biophys Acta 2008; 1777: 1-14]. Within the tumor microenvironment, the fast growth rate of cancer cells combined with an often malformed and defective vasculature often results in hypoxic regions with oxygen levels ranging from 0-5%. Given the effects of oxygen levels on energy metabolism [Xie et al., J Biol Chem 2017; 292: 16825-16832], and to further mimic the physiological conditions *in vivo,* the growth of the cancer cell lines in Medium A, B and C was studied at both ambient, 21% O₂, and at the more physiological 5% O₂.

### Ketones

The ketone bodies acetoacetate (Acac), BOHB and acetone are produced by the liver during fasting or starvation. BOHB is the major ketone body in mammals, while Acac constitutes around 20%. The majority of published *in vitro* studies where the effect of ketones on cancer cells is studied mainly focus on BOHB, however there are studies that suggest different effects from the addition of Acac compared to BOHB [Vallejo et al., J Neurooncol 2020; 147: 317-326]. To further mimic the *in vivo* ketogenic situation where both ketones are present, and to investigate a possible differential effect of BOHB and Acac, Acac was also included in the study.

### Materials and Methods

### Cell lines

All cell lines were purchased from ATCC (ATCC, LGC standards) except for RCC4 that was from Sigma-Aldrich (Merck). Primary human renal cell carcinoma cells were isolated from nephrectomies performed at Sahlgrenska University Hospital in Gothenburg, Sweden, after informed patient consent and with permit from the regional ethical committee. Optimal seeding density was determined for each cell line in 96-well plates cultured for 3 days in standard cell culture medium.

### Culture conditions and additives

Cells were maintained in RPMI-1640 medium (31870-025 GIBCO) with the addition of 10% serum, 200 mM L-glutamine and 1% penicillin-streptomycin (PEST) in humidified chambers at 37°C and 5% CO₂. For hypoxic conditions (5% O₂), cells were maintained in a Galaxy 14 S CO₂ incubator (Eppendorf) where N₂ was used to adjust the O₂ level to 5%.

Medium A, B, and C were prepared as follows.

Medium A: RPMI1640 (31870-025, GIBCO) with the addition of 1% PEST, 200 mM L-glutamine and 10% dialyzed serum. Dialyzed serum was used to reduce the amounts of small molecules such as amino acids.

Medium B and C were prepared from RPMI1640 modified medium powder without L-glutamine, glucose and amino acids (R9010-01, US Biological Life Sciences). For 1L medium, 7.4g powder was dissolved in 900 ml sterile water without heating and 2g sodium bicarbonate was added. Amino acids listed in Table 5 were added to the same concentration as in full RPMI1640 medium (Table 5). After all additions, the medium was sterilized by filtering through 0.22um membranes and divided into two bottles.

In Medium B, to mimic physiological conditions, the levels of glutamine, serine, glycine, arginine and glucose were set to the median of measured levels in human serum, based on data from the Mayo Clinic Laboratories (https://www.mayocliniclabs.com/test-catalog/Clinical+and+Interpretive/9265).

To model Cancer Dialysis conditions in Medium C, the levels of these nutrients were reduced to 50% of the physiological levels. As for Medium A, 1% PEST and 10% dialyzed serum were added to Medium B and C. The concentrations of the selected nutrients in Medium A-C are summarized in Table 6. Sodium pyruvate, that is a common addition in cell culture medium, was not present in any of the used media.

**Table 5. Amino acid concentrations in RPMI1640**

| **Amino Acids** | **(g/L)** | **dissolved in** |
|---|---|---|
| L-Asparagine | 0.0568 | H₂O |
| L-Aspartic Acid | 0.02 | 1M HCl |
| L-Cystine• | 0.065 | 2M HCl |
| L-Glutamic Acid | 0.02 | 1M HCl |
| L-Histidine | 0.02 | H₂O |
| Hydroxy-L-proline | 0.02 | H₂O |
| L-Isoleucine | 0.05 | 1M HCl |
| L-Leucine | 0.05 | 1M HCl |
| L-Lysine | 0.04 | H₂O |
| L-Methionine | 0.015 | H₂O |
| L-Phenylalanine | 0.015 | 1M HCl |
| L-Proline | 0.02 | H₂O |
| L-Threonine | 0.02 | H₂O |
| L-Tryptophan | 0.005 | 1M HCl |
| L-Tyrosine | 0.024 | 1M HCl |
| L-Valine | 0.02 | H₂O |

**Table 6. Nutrient composition of Medium A, B and C.**

| | **Medium A RPMI-1640** | **Medium B physiological** | **Medium C cancer dialysis** |
|---|---|---|---|
| Glucose (mM) | 11 | 5.85 | 2.93 |
| BOHB (mM) | 0 | 0 | 8 |
| Glutamine (uM) | 2000 | 664 | 332 |
| Serine (uM) | 280 | 125 | 62.5 |
| Glycine (uM) | 130 | 308 | 154 |
| Arginine (uM) | 1150 | 76 | 38 |

Amino acids and other additives were purchased from Sigma Aldrich.

After all nutrients were added pH was measured. The pH values were as follows: Full RPMI1640 with 10% non-dialyzed FBS, 1% PEST and 200mM L-glutamine, pH 7.78; Medium A, pH 7.56; Medium B, pH 7.62 and Medium C, pH 7.57.

Stock solutions of DL-β-Hydroxybutyric acid sodium salt (H6501, Sigma Aldrich) and Lithium Acetoacetate (A8509, Sigma Aldrich) were prepared in water, sterile filtered, aliquoted and stored at -20°C. Lithium Chloride (L7026, Sigma Aldrich) was used as a control for the addition of Lithium in the Li-Acac.

### Viability assay

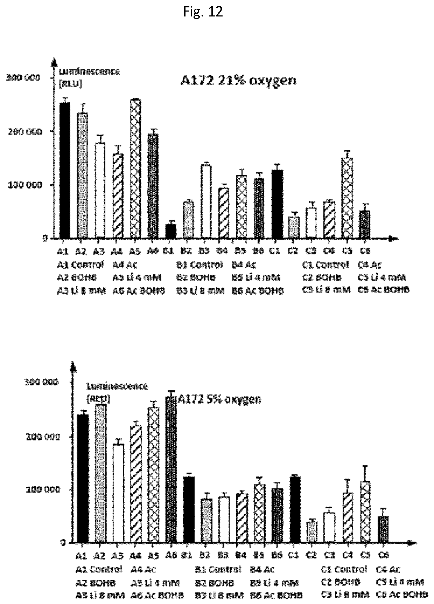

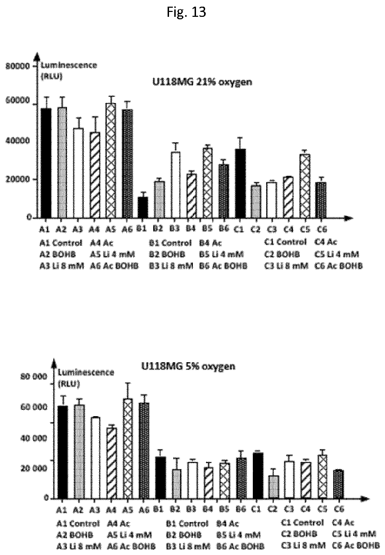

CellTiter-Glo Luminescent cell viability assay (Promega) was used as a readout of cell numbers according to the manufacturers' instructions. In experiments shown in Figure 12-13, double plates were seeded and treated. One set of plates was used for collection of medium for lactate measurement (see below) and the CellTiterGlo-assay. The other set of plates was frozen at -80°C at the end of the experiment. The frozen plates were intended for the CyQuant cell proliferation assay (Thermo Fisher) that measures the amount of DNA per well. Analysis of the amounts of cells by both CellTiterGlo and CyQuant assays would ensure that effects of the culture conditions on viability or growth rate would not be concealed by simultaneous changes in ATP-levels per cell.

### Collection of medium for lactate measurement

In experiments shown in Figure 12-13, cell culture medium was collected on day 3, transferred to new 96-well plates and frozen at -80°C. This medium could be used to analyze the amount of excreted lactate as a measurement of metabolic state. Several kits for lactate measurement are available, for example the Lactate-Glo assay (J5021, Promega) is designed for use in assays where serum is present.

### RESULTS

Example 2 was designed to answer the following question:
- Is the growth of the selected cancer cell lines affected by the Cancer Dialysis conditions emulated in Medium C at normoxia or hypoxia?

### Growth in Medium A, B and C

As a first step, Medium A-C were prepared as described in Materials and methods, and the ability of the cancer cell lines to grow in these media were tested. Growth curves for the selected cell lines over time in each medium were established. The number of cells was analyzed after 1, 2 and 3 days of culture in medium A-C at normoxia (21% O₂).

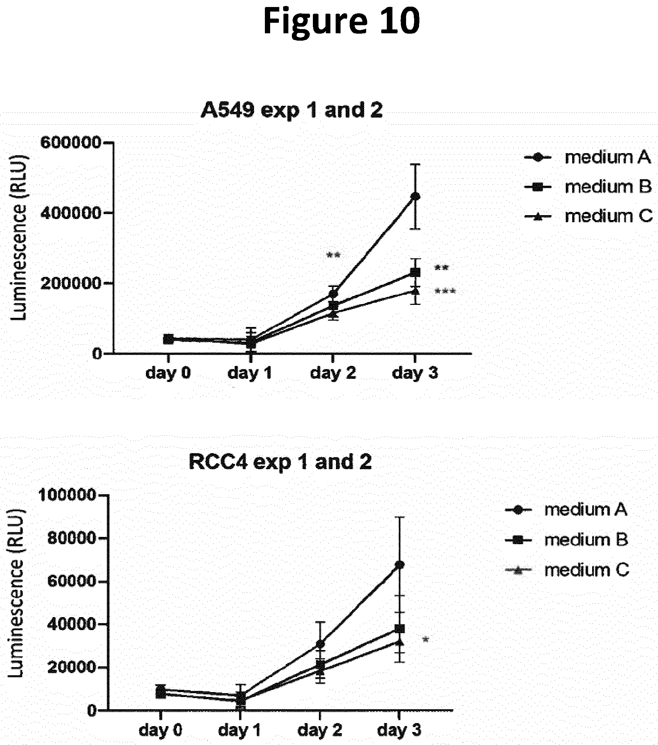

As shown in **Figure 10****,** reduction of the selected nutrients to more physiological levels as in Medium B reduced the growth rate of A549 lung carcinoma and RCC4 renal carcinoma cell lines significantly compared to Medium A. Medium C further reduced growth rates compared to Medium A.

### Growth of cancer cells in Medium A-C at normoxia and hypoxia

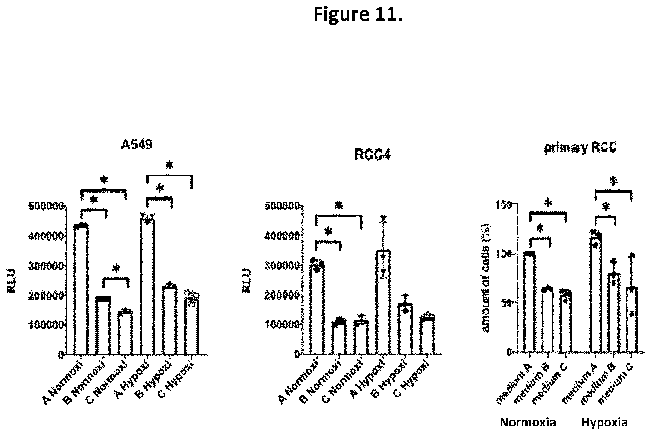

In **Figure 11****,** the amounts of cells after 3 days of culture in Medium A-C in normoxia and hypoxia are shown for the A549 lung carcinoma and RCC4 renal carcinoma cell lines as well as for primary renal carcinoma (RCC) cells. Again, the growth rate at normoxia was reduced in Medium B and C compared to Medium A. The same pattern was found in cells cultured at 5% O₂. Again, in RCC4, the low nutrient levels and addition of BOHB in Medium C did not have a significant additional effect compared to the conditions in Medium B.

For A549, a small but significant reduction in growth rate was found between Medium B and C, but only at normoxia.

Primary renal carcinoma cells from three patients were included in this study. Similar to the established cell lines, these cells showed a reduced growth rate in Medium B and C compared to Medium A.

Overall, changing the oxygen pressure from 21% (normoxia) to 5% O₂ (hypoxia) had very limited effect on the growth rate of these cells.

Next, it was decided to also include Acac in the study and analyze the viability of cells cultured in the presence of BOHB and Acac alone or in combination in Medium A-C. The experiment was performed at 21% and 5% O₂. The experiments were made with glioma cell lines A172 and U118MG.

Being a chiral molecule, BOHB exists as two enantiomers, D- and L-BOHB. D-BOHB is normally produced and metabolized in humans. The BOHB-salt used in this study contains a mixture of 50:50 of D- and L-BOHB. In order to ensure a high presence of the active D-form, the total concentration of BOHB added was increased to 16mM, giving a level of 8mM D-BOHB. To keep the total concentration of active ketones constant, 8mM Acac was used, and for the combination of both ketones, the levels were adjusted to 4mM Acac and 8mM BOHB (containing 4mM D-BOHB).

The Acac available for *in vitro* use is in the form of a lithium salt. Since lithium itself can affect the viability of cancer cells [Cohen-Harazi et al., Anticancer Res 2020; 40: 3831-3837], 8mM LiCl was used as a control for the 8mM Acac data point and 4mM LiCl was used as a control for the 4mM Acac/8mM BOHB data.

At the end of the experiment, the culture media from each well was collected and frozen to enable later determination of lactate levels as a measurement of the metabolic state. In addition, double experiments were performed, where one set of plates were frozen for later quantification of cell numbers using the CyQuant proliferation assay, and the amounts of cells in the other set were analyzed by CellTiterGlo viability assay.

### The effect of BOHB and Acac added alone or in combination

As shown in **Figure 12-13****,** the initial experiment gave promising data regarding the effect of high ketone concentration in nutrient reduced conditions on the growth of A172 and U118MG glioma cell lines.

In Medium A, at both normoxia and hypoxia, addition of 16mM BOHB alone had no growth inhibitory effect in A172 or U118MG cells, and 8mM Acac did not reduce the number of cells further compared to the 8mM LiCl control.

However, addition of 4mM Acac in combination with 8mM BOHB significantly reduced the number of A172 cells in Medium A at normoxia compared to the 4mM LiCl control.

The interpretation of the results from Medium B was disturbed by a technical error in the normoxic control sample. However, in hypoxic A172 cells, BOHB significantly reduced the number of cells to approximately 70% of the amount in Medium B without BOHB. A similar reduction was also seen in hypoxic U118MG cells.

Furthermore, in Medium B, 8mM Acac reduced the number of cells significantly compared to the 8mM LiCl control in both cell lines, but only at 21%O₂.

In Medium C, addition of 8mM Acac alone did not significantly reduce the amounts of cells compared to the 8mM LiCl control. However, in both cell lines and at both 21 and 5% O₂, addition of 16mM BOHB significantly reduced the number of cells to around 30% compared to C-medium without BOHB.

Also the combination of BOHB and Acac resulted in significantly fewer cells in Medium C compared to the 4mM LiCl control, in both cell lines and at both oxygen levels.

These results suggest that addition of high levels of BOHB or Acac alone do not inhibit the growth of glioma cells in a nutrient rich environment such as Medium A. Also in Medium B, with more physiological nutrient levels, only small differences were seen when the ketones were added. The largest effects were found in Medium C, where both 16mM BOHB alone and the combination of 8mM BOHB with 4mM Acac significantly reduced the number of cells compared to their respective controls, at both hypoxia and normoxia. This was not seen when 8mM Acac was added alone.

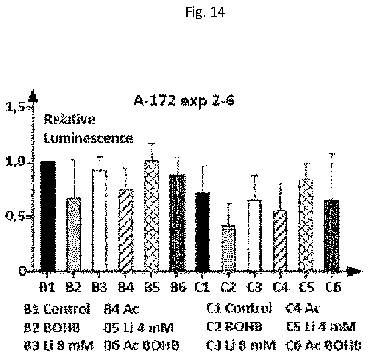

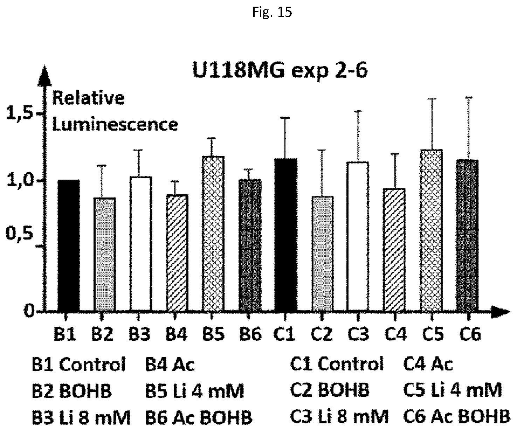

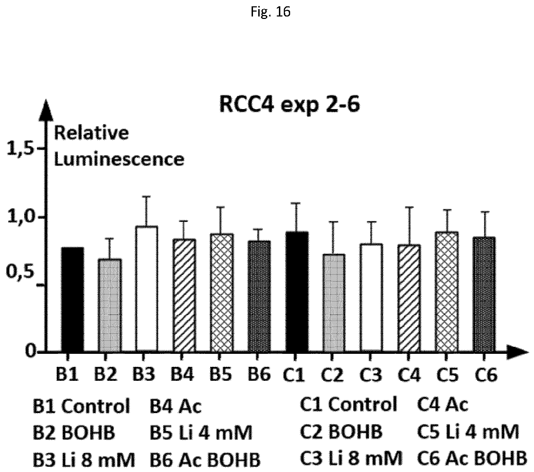

However, repetition of these experiments, with focus on Medium B and C at normoxic conditions, gave inconsistent results. **Figures 14-16** show the combined results from experiment 2-6, for the glioma cell lines A172 and U118MG and the renal carcinoma cell line RCC4. A trend of reduced growth was seen in Medium C when BOHB or Acac were added separately, especially in the A172 cell line.

The results from the first and second parts of Example 2 indicated that the cancer cell lines grew slower in a nutrient restricted environment. The tested cell lines seemed viable in Medium B and C although the proliferation rate was reduced. Optical inspection of the cells at day 3 did not reveal any floating cells, which could have been a sign of dead cells.

The data from the third part of the study shows an increased sensitivity of glioma cell lines to high levels of BOHB or a combination of BOHB and Acac in the nutrient restricted Medium C. Such a sensitivity was, however, not found for the renal carcinoma cell line RCC4.

## Claims

1. An extracorporeal blood treatment system (50) for treating a subject suffering from cancer, the system comprising:
an extracorporeal blood circuit (52a, 52b);
a dialysate fluid circuit (54a, 54b);
said extracorporeal blood circuit (52a, 52b) and dialysate fluid circuit (54a, 54b) being divided by a membrane (59) of a filtration unit (58);
at least one blood pump (60) for controlling the flow of blood through the blood circuit (52a, 52b);
at least one dialysate fluid pump (62, 68) for controlling the flow of dialysate fluid through the dialysate fluid circuit (54a, 54b);
optionally one or more infusion lines (66, 80, 81, 82), each infusion line being connected to the extracorporeal blood circuit (52a, 52b) or adapted for being directly connected to the vascular system of the subject to be treated, each infusion line comprising an infusion pump;
a system computing unit (64) operatively connected to the blood pump (60) and the dialysate fluid pump (62, 68) and optionally to the one or more infusion pumps of the one or more infusion lines (66, 80,81, 82), said system computing unit having a user interface including an input means and a display means; wherein
the system computing unit (64) is adapted for receiving a desired blood concentration value GLN_{b} of glutamine (901) within the range of 0.1 and 0.5 mM;
the system computing unit (64) is adapted for receiving a desired blood concentration value GLUCOSE_{b} of glucose (903) within the range of 2 to 4 mM;
the system computing unit (64) is adapted for receiving a desired blood concentration value KETONE_{b} of a ketone body, such as acetoacetate, beta-hydroxybutyrate or
pharmaceutically acceptable derivatives, esters and salts thereof (905) within the range of 1 - 15 mM;
the system computing unit (64) is adapted for receiving a concentration value GLNₚ representing the concentration of glutamine or pharmaceutically acceptable glutamine-containing compounds in fresh dialysate fluid (902);
the system computing unit (64) is adapted for receiving a concentration value GLUCOSEₚ representing the concentration of glucose in fresh dialysate fluid ₍904);
optionally, the system computing unit (64) is adapted for receiving a concentration value KETONEₚ,,representing the concentration of a ketone body, such as acetoacetate, beta-hydroxybutyrate or pharmaceutically acceptable derivatives, esters and salts thereof in fresh dialysate fluid (907);
optionally, the system computing unit (64) is adapted for receiving a concentration value KETONEᵢ representing the concentration of a ketone body such as acetoacetate,
beta-hydroxybutyrate or pharmaceutically acceptable derivatives, esters and salts thereof in an infusion liquid to be infused into the extracorporeal blood line (52b) or
directly into the vascular system of said subject to be treated, through one of said one or more infusion lines (66, 80, 81, 82) (906);
the system computing unit (64) is adapted for receiving an actual concentration value GLNₐ of glutamine in blood from said treated subject (909) and receiving an actual concentration value GLUCOSEₐ of glucose in blood from said treated subject (910) and
receiving an actual concentration value KETONEₐ of a ketone body such as acetoacetate and/or beta-hydroxybutyrate (911);
the system computing unit (64) being adapted for controlling said blood pump (60) and said dialysate fluid pump (62, 68) in such a way that the actual blood concentration value GLNₐ of glutamine is driven towards or below GLN_{b} (912) and the actual blood concentration value GLUCOSEₐ of glucose is driven towards or below GLUCOSE_{b} (914);
and
if the system (50) comprises one or more of said infusion lines (66, 80, 81, 82) and in case one of said infusion lines (66, 80, 81, 82) is adapted for infusing said infusion liquid into the extracorporeal blood line (52b) or directly into the vascular system of said subject to be treated, the system computing unit (64) is adapted for controlling said infusion pump of said infusion line in such a way that the actual blood concentration value KETONEₐ is driven towards KETONE_{b} (920); or alternatively
if the system (50) does not comprise such an infusion line (66, 80, 81, 82), the system computing unit (64) is adapted for comparing KETONEₐ and KETONE_{b}, and if KETONEₐ < KETONE_{b}, displaying a message on said display informing that the treated subject should consume a further amount of ketone bodies or medium chain triglycerides.

2. An extracorporeal blood treatment system (50) according to claim 1, wherein the system (50) comprises one or more of said infusion lines (66, 80, 81, 82), and the system computing unit (64) is adapted for receiving a concentration value KETONEᵢ representing the concentration of a ketone body such as acetoacetate, beta-hydroxybutyrate or pharmaceutically acceptable derivatives, esters and salts thereof in an infusion liquid to be infused into the extracorporeal blood line (52b) or directly into the vascular system of the subject to be treated, through one of said one or more infusion lines (66, 80, 81, 82) (906), and
the system computing unit (64) is adapted for controlling said infusion pump of said infusion line in such a way that the actual blood concentration value KETONEₐ is driven towards KETONE_{b} (920).

3. An extracorporeal blood treatment system (50) according to claim 2, wherein the system computing unit (64) is adapted for monitoring GLNₐ and initiating infusion of a composition containing glutamine or pharmaceutically acceptable glutamine-containing compounds if GLNₐ is lower than GLN_{b} (916) by starting a relevant infusion pump in one of the one or more infusion lines (66, 80, 81, 82), and maintaining said infusion until GLNₐ is equal to GLN_{b}.

4. An extracorporeal blood treatment system (50) according to any one of claims 2 to 3, wherein the system computing unit (64) is adapted for monitoring GLUCOSEₐ, and initiating infusion of a composition containing glucose if GLUCOSEₐ is lower than GLUCOSE_{b} (918) by starting a relevant infusion pump in one of the one or more infusion lines (66, 80, 81, 82), and maintaining said infusion until GLUCOSEₐ is equal to GLUCOSE_{b} (918).

5. A system according to any preceding claim wherein the filtration membrane has a molecular weight cut off (MWCO) of less than 60kDa.

6. A system according to claim 5 wherein the filtration membrane has a MWCO of less than about 50kDa or less than about 40 kDa, less than 30 kDa, less than 10 kDa, less than 5 kDa or less than 2 kDa.

7. A system according to any preceding claim wherein the blood circuit comprises a thermal management system for heating or cooling blood in the blood line during use.

8. A system according to claim 7 wherein the thermal management system is controllable to regulate the temperature of blood in the blood circuit to a temperature between 20°C and 43°C.

9. A system according to any preceding claim, further comprising on or more sensors (S, 90, 91, 92, 93) for the detection of analytes selected from the group glucose, glutamine, and ketone bodies, the sensor(s) (S, 90, 91, 92, 93) being preferably positioned in an effluent portion (54a) of the dialysate fluid circuit, the sensors being in communication with the system processing unit (64) and providing an output indicative of the concentration of the analyte(s) in blood or preferably in spent dialysate fluid; wherein the system processing unit (64) is configured to determine from the output(s) of the sensor(s) (S, 90, 91, 92, 93) a representative blood concentration of the analyte(s), thereby monitoring at least one of GLNₐ, GLUCOSEₐ and KETONEₐ.

10. A therapy set for use in an extracorporeal blood treatment system (50) according to any of claims 1 - 9, the set comprising
a filtration unit (58) having a membrane (59) which divides an integrated blood line (52a, 52b) and an integrated dialysate fluid line (54a, 54b), wherein the blood line (52a, 52b) and/or the dialysate fluid line (54a, 54b) comprises sensors (S, 90, 91, 92, 93) for monitoring at least one of GLNₐ, GLUCOSEₐ, and KETONEₐ.

11. A system computing unit (64) adapted for controlling an extracorporeal blood treatment system (50) for treating a subject suffering from cancer; said system computing unit comprising:
a plurality of output means adapted for being operatively connected to at least one blood pump (60), at least one dialysate fluid pump (62, 68) and optionally one or more infusion pumps for controlling the flow in each of one or more infusion lines (66, 80, 81, 82);
a user interface including an input means, and a display means; and
a memory means and a calculation means;
the system computing unit (64) being adapted for receiving a desired blood concentration value GLN_{b} of glutamine (901);
the system computing unit (64) being adapted for receiving a desired blood concentration value GLUCOSE_{b} of glucose (903);
the system computing unit (64) being adapted for receiving a desired blood concentration value KETONE_{b} of a ketone body (905)
the system computing unit (64) being adapted for receiving a dialysate concentration value GLNₚ of glutamine (902);
the system computing unit (64) being adapted for receiving a dialysate concentration value GLUCOSEₚ of glucose (904);
optionally, the system computing unit (64) being adapted for receiving a concentration value KETONEₚ,,representing the concentration of a ketone body, such as acetoacetate, beta-hydroxybutyrate or pharmaceutically acceptable derivatives, esters and salts thereof in fresh dialysate fluid (907);
optionally, the system computing unit (64) being adapted for receiving an infusion liquid concentration value KETONEᵢ of a ketone body (906);
the system computing unit (64) being adapted for receiving an actual blood concentration value GLNₐ of glutamine (909);
the system computing unit (64) being adapted for receiving an actual blood concentration value GLUCOSEₐ of glucose (910);
the system computing unit (64) being adapted for receiving an actual blood concentration value KETONEₐ of a ketone body such as beta-hydroxybutyrate and/or acetoacetate (911);
the system computing unit (64) being adapted for controlling said blood pump (60) and said dialysate fluid pump (62, 68) in such a way that the actual blood concentration value GLNₐ of glutamine is driven towards or below GLN_{b} (912), and the actual blood concentration value GLUCOSEₐ of glucose is driven towards or below Glucose_{b} (914); and
in case an infusion pump is operatively connected to the system computing unit (64), the system computing unit (64) is adapted for controlling the infusion pump in such a way that the actual blood concentration value KETONEₐ is driven towards KETONE_{b} (920);
and
in case no infusion pump is operatively connected to the system computing unit (64), the system computing unit (64) is adapted for comparing KETONEₐ and KETONE_{b}, and if KETONEₐ < KETONE_{b}, displaying a message on said display informing that the treated subject should consume a further amount of ketone bodies or medium chain triglycerides.

12. A dialysis fluid, comprising ketone bodies such as acetoacetate, beta-hydroxybutyrate or pharmaceutically acceptable derivatives, esters and salts thereof, for use in a dialysis therapy method of treating cancer.

13. A dialysis fluid for use in a dialysis therapy method of treating cancer according to claim 12, further comprising at least one of
a) glutamine or glutamine-containing compounds; and
b) glucose.

14. A dialysis fluid for use in a dialysis therapy method treating of cancer according to claim 12, wherein the concentration of:
a) glutamine or glutamine-containing compounds amounts to 0 - 0.5 mM, and preferably 0.05 - 0.3 mM;
b) glucose amounts to 0 - 6 mM and preferably 0.5 - 4 mM; and
c) ketone bodies amount to 1 - 15 mM and preferably 2 - 12 mM.

## Patentansprüche

1. Extrakorporales Blutbehandlungssystem (50) zur Behandlung eines an Krebs leidenden Subjekts, wobei das System umfasst:
einen extrakorporalen Blutkreislauf (52a, 52b);
einen Dialysatfluidkreislauf (54a, 54b);
wobei der extrakorporale Blutkreislauf (52a, 52b) und der Dialysatfluidkreislauf (54a, 54b) durch eine Membran (59) einer Filtrationseinheit (58) geteilt sind;
mindestens eine Blutpumpe (60) zum Steuern des Blutflusses durch den Blutkreislauf (52a, 52b);
mindestens eine Dialysatfluidpumpe (62, 68) zum Steuern des Flusses von Dialysatfluid durch den Dialysatfluidkreislauf (54a, 54b);
gegebenenfalls eine oder mehrere Infusionsleitungen (66, 80, 81, 82), wobei jede Infusionsleitung mit dem extrakorporalen Blutkreislauf (52a, 52b) verbunden ist oder eingerichtet ist, um direkt mit dem Gefäßsystem des zu behandelnden Subjekts verbunden zu werden, wobei jede Infusionsleitung eine Infusionspumpe umfasst;
eine Systemrecheneinheit (64), die operativ mit der Blutpumpe (60) und der Dialysatfluidpumpe (62, 68) und
gegebenenfalls mit der einen oder den mehreren Infusionspumpen der einen oder mehreren Infusionsleitungen (66, 80, 81, 82) verbunden ist, wobei die Systemrecheneinheit eine Benutzerschnittstelle aufweist, die ein Eingabemittel und ein Anzeigemittel aufweist; wobei
die Systemrecheneinheit (64) eingerichtet ist, um einen gewünschten Blutkonzentrationswert GLN_{b} von Glutamin (901) innerhalb des Bereichs von 0,1 und 0,5 mm zu empfangen;
die Systemrecheneinheit (64) eingerichtet ist, um einen gewünschten Blutkonzentrationswert GLUCOSE_{b} von Glucose innerhalb des Bereichs von 2 bis 4 mM zu empfangen (903);
die Systemrecheneinheit (64) eingerichtet ist, um einen gewünschten Blutkonzentrationswert KETONE_{b} eines Ketonkörpers, wie Acetoacetat, beta-Hydroxybutyrat oder
pharmazeutisch verträgliche Derivate, Ester und Salze davon, im Bereich von 1 - 15 mm zu empfangen (905);
die Systemrecheneinheit (64) eingerichtet ist, um einen Konzentrationswert GLN_{P}, der die Konzentration von Glutamin oder pharmazeutisch verträglichen Glutamin enthaltenden Verbindungen in frischem Dialysatfluid repräsentiert, zu empfangen (902);
die Systemrecheneinheit (64) eingerichtet ist, um einen Konzentrationswert GLUCOSEₚ, der die Konzentration von Glucose in frischem Dialysatfluid repräsentiert, zu empfangen (904);
wobei die Systemrecheneinheit (64) gegebenenfalls eingerichtet ist, um einen Konzentrationswert KETONEₚ,
der die Konzentration eines Ketonkörpers, wie Acetoacetat, Beta-Hydroxybutyrat oder pharmazeutisch verträgliche Derivate, Ester und Salze davon, in frischem Dialysatfluid repräsentiert, zu empfangen (907);
wobei die Systemrecheneinheit (64) gegebenenfalls eingerichtet ist, um einen Konzentrationswert KETONEᵢ,
der die Konzentration eines Ketonkörpers, wie Acetoacetat, beta-Hydroxybutyrat oder pharmazeutisch verträgliche Derivate, Ester und Salze davon, in einer Infusionsflüssigkeit repräsentiert, die durch eine der einen oder mehreren Infusionsleitungen (66, 80, 81, 82) in die extrakorporale Blutleitung (52b) oder direkt in das Gefäßsystem des zu behandelnden Subjekts infundiert werden soll, zu empfangen (906);
wobei die Systemrecheneinheit (64) eingerichtet ist, um einen tatsächlichen Konzentrationswert GLNₐ von Glutamin in Blut von dem behandelten Subjekt zu empfangen (909),
und einen tatsächlichen Konzentrationswert GLUCOSEₐ von Glucose in Blut von dem behandelten Subjekt (910) zu empfangen, und einen tatsächlichen Konzentrationswert KETONEₐ eines Ketonkörpers, wie Acetoacetat und/oder beta-Hydroxybutyrat, zu empfangen (911);
wobei die Systemrecheneinheit (64) eingerichtet ist, um die Blutpumpe (60) und die Dialysatfluidpumpe (62, 68) so zu steuern, dass der tatsächliche Blutkonzentrationswert GLNₐ von Glutamin zu oder unter GLN_{b} (912) getrieben wird, und der tatsächliche Blutkonzentrationswert GLUCOSEₐ von Glucose zu oder unter GLUCOSE_{b} getrieben wird (914);
und
falls das System (50) eine oder mehrere der Infusionsleitungen (66, 80, 81, 82) umfasst, und wenn eine der Infusionsleitungen (66, 80, 81, 82) eingerichtet ist, um die Infusionsflüssigkeit in die extrakorporale Blutleitung (52b) oder direkt in das Gefäßsystem des zu behandelnden Subjekts zu infundieren, die Systemrecheneinheit (64) eingerichtet ist, um die Infusionspumpe der Infusionsleitung so zu steuern, dass der tatsächliche Blutkonzentrationswert KETONEₐ in Richtung KETONE_{b} (920) getrieben wird; oder alternativ falls das System (50) keine solche Infusionsleitung (66, 80, 81, 82) umfasst, die Systemrecheneinheit (64) eingerichtet ist, um KETONEₐ und KETONE_{b} zu vergleichen, und falls KETONEₐ < KETONE_{b}, eine Nachricht auf der Anzeige anzuzeigen, die informiert, dass das behandelte Subjekt eine weitere Menge an Ketonkörpern oder mittelkettigen Triglyceriden verbrauchen sollte.

2. Extrakorporales Blutbehandlungssystem (50) nach Anspruch 1, wobei
das System (50) eine oder mehrere der Infusionsleitungen (66, 80, 81, 82) umfasst, und
die Systemrecheneinheit (64) eingerichtet ist, um einen Konzentrationswert KETONEᵢ, der die Konzentration eines Ketonkörpers, wie Acetoacetat, beta-Hydroxybutyrat oder pharmazeutisch verträgliche Derivate, Ester und Salze davon, in einer Infusionsflüssigkeit repräsentiert, die über eine der einen oder mehreren Infusionsleitungen (66, 80, 81, 82) in die extrakorporale Blutleitung (52b) oder direkt in das Gefäßsystem des zu behandelnden Subjekts zu infundieren ist, zu empfangen (906), und
die Systemrecheneinheit (64) eingerichtet ist, um die Infusionspumpe der Infusionsleitung so zu steuern, dass der tatsächliche Blutkonzentrationswert KETONEₐ in Richtung KETONE_{b} getrieben wird (920).

3. Extrakorporales Blutbehandlungssystem (50) nach Anspruch 2, wobei die Systemrecheneinheit (64) eingerichtet ist, um GLNₐ zu überwachen und eine Infusion einer Glutamin oder pharmazeutisch verträgliche Glutamin enthaltende Verbindungen enthaltenden Infusion zu initiieren, falls GLNₐ niedriger als GLN_{b} ist (916), indem eine relevante Infusionspumpe in einer der einen oder mehreren Infusionsleitungen (66, 80, 81, 82) gestartet wird und die Infusion beibehalten wird, bis GLNₐ gleich GLN_{b} ist.

4. Extrakorporales Blutbehandlungssystem (50) nach einem der Ansprüche 2 bis 3, wobei die Systemrecheneinheit (64) eingerichtet ist, um GLUCOSEₐ zu überwachen und die Infusion einer Glucose enthaltenden Zusammensetzung zu initiieren, falls GLUCOSEₐ niedriger als GLUCOSE_{b} ist (918), indem eine relevante Infusionspumpe in einer der einen oder mehreren Infusionsleitungen (66, 80, 81, 82) gestartet wird und die Infusion beibehalten wird, bis GLUCOSE_{b} gleich GLUCOSE_{b} ist (918).

5. System nach einem der vorhergehenden Ansprüche, wobei die Filtrationsmembran eine Molekulargewichtsausschlussgrenze (MWCO) von weniger als 60 kDa aufweist.

6. System nach Anspruch 5, wobei die Filtrationsmembran eine MWCO von weniger als etwa 50 kDa oder weniger als etwa 40 kDa, weniger als 30 kDa, weniger als 10 kDa, weniger als 5 kDa oder weniger als 2 kDa aufweist.

7. System nach einem der vorhergehenden Ansprüche, wobei der Blutkreislauf ein Wärmemanagementsystem zum Erwärmen oder Kühlen von Blut in der Blutleitung während der Verwendung umfasst.

8. System nach Anspruch 7, wobei das Wärmemanagementsystem steuerbar ist, um die Temperatur von Blut in dem Blutkreislauf auf eine Temperatur zwischen 20 °C und 43 °C zu regulieren.

9. System nach einem der vorhergehenden Ansprüche, ferner umfassend einen oder mehrere Sensoren (S, 90, 91, 92, 93) zur Detektierung von Analyten ausgewählt aus der Gruppe Glucose, Glutamin und Ketonkörper, wobei der oder die Sensor(en) (S, 90, 91, 92, 93) vorzugsweise in einem Ausflussteil (54a) des Dialysatfluidkreislaufs positioniert ist/sind, wobei die Sensoren in Kommunikation mit der Systemverarbeitungseinheit (64) stehen und eine Ausgabe bereitstellen, die die Konzentration des oder der Analyten in Blut oder vorzugsweise in verbrauchtem Dialysatfluid angibt; wobei die Systemverarbeitungseinheit (64) dazu ausgelegt ist, aus der oder den Ausgabe(n) des oder der Sensoren (S, 90, 91, 92, 93) eine repräsentative Blutkonzentration des oder der Analyten zu bestimmen, wodurch mindestens eines von GLNₐ, GLUCOSEₐ und KETONEₐ überwacht wird.

10. Therapieset zur Verwendung in einem extrakorporalen Blutbehandlungssystem (50) nach einem der Ansprüche 1 - 9, wobei das Set umfasst:
eine Filtrationseinheit (58) mit einer Membran (59), die eine integrierte Blutleitung (52a, 52b) und eine integrierte Dialysatfluidleitung (54a, 54b) teilt, wobei die Blutleitung (52a, 52b) und/oder die Dialysatfluidleitung (54a, 54b) Sensoren (S, 90, 91, 92, 93) zur Überwachung von GLNₐ, GLUCOSEₐ und/oder KETONEₐ umfasst.

11. Systemrecheneinheit (64), die eingerichtet ist, um ein extrakorporales Blutbehandlungssystem (50) zum Behandeln eines an Krebs leidenden Subjekts zu steuern; wobei die Systemrecheneinheit umfasst:
eine Vielzahl von Ausgabemitteln, die eingerichtet ist, um operativ mit mindestens einer Blutpumpe (60), mindestens einer Dialysatfluidpumpe (62, 68) und gegebenenfalls einer oder mehreren Infusionspumpen zum Steuern des Flusses in jeder von einer oder mehreren Infusionsleitungen (66, 80, 81, 82) verbunden zu sein;
eine Benutzerschnittstelle, die ein Eingabemittel und ein Anzeigemittel einschließt; und
ein Speichermittel und ein Berechnungsmittel;
wobei die Systemrecheneinheit (64) eingerichtet ist, um einen gewünschten Blutkonzentrationswert GLN_{b} von Glutamin zu empfangen (901);
wobei die Systemrecheneinheit (64) eingerichtet ist, um einen gewünschten Blutkonzentrationswert GLUCOSE_{b} von Glucose zu empfangen (903);
wobei die Systemrecheneinheit (64) eingerichtet ist, um einen gewünschten Blutkonzentrationswert KETONE_{b} eines Ketonkörpers zu empfangen (905);
wobei die Systemrecheneinheit (64) eingerichtet ist, um einen Dialysatkonzentrationswert GLNₚ von Glutamin zu empfangen (902);
wobei die Systemrecheneinheit (64) eingerichtet ist, um einen Dialysatkonzentrationswert GLUCOSEₚ von Glucose zu empfangen (904);
wobei die Systemrecheneinheit (64) gegebenenfalls eingerichtet ist, um einen Konzentrationswert KETONEₚ, der die Konzentration eines Ketonkörpers, wie Acetoacetat, Beta-Hydroxybutyrat oder pharmazeutisch verträgliche Derivate, Ester und Salze davon, in frischem Dialysatfluid repräsentiert, zu empfangen (907);
wobei die Systemrecheneinheit (64) gegebenenfalls eingerichtet ist, um einen Infusionsflüssigkeitskonzentrationswert KETONEᵢ eines Ketonkörpers (906) zu empfangen;
wobei die Systemrecheneinheit (64) eingerichtet ist, um einen tatsächlichen Blutkonzentrationswert GLNₐ von Glutamin zu empfangen (909);
wobei die Systemrecheneinheit (64) eingerichtet ist, um einen tatsächlichen Blutkonzentrationswert GLUCOSEₐ von Glucose zu empfangen (910);
wobei die Systemrecheneinheit (64) eingerichtet ist, um einen tatsächlichen Blutkonzentrationswert KETONEₐ eines Ketonkörpers, wie Beta-Hydroxybutyrat und/oder Acetoacetat, zu empfangen (911);
wobei die Systemrecheneinheit (64) eingerichtet ist, um die Blutpumpe (60) und die Dialysatfluidpumpe (62, 68) so zu steuern, dass der tatsächliche Blutkonzentrationswert GLNₐ von Glutamin zu oder unter GLN_{b} (912) getrieben wird, und der tatsächliche Blutkonzentrationswert GLUCOSEₐ von Glucose zu oder unter Glucose_{b} getrieben wird (914); und
falls eine Infusionspumpe operativ mit der Systemrecheneinheit (64) verbunden ist, die Systemrecheneinheit (64) eingerichtet ist, um die Infusionspumpe so zu steuern, dass der tatsächliche Blutkonzentrationswert KETONEₐ in Richtung KETONE_{b} getrieben wird (920);
und
falls keine Infusionspumpe operativ mit der Systemrecheneinheit (64) verbunden ist, die Systemrecheneinheit (64) eingerichtet ist, um KETONEₐ und KETONE_{b} zu vergleichen, und falls KETONEₐ < KETONE_{b}, eine Nachricht auf der Anzeige anzuzeigen, die informiert, dass das behandelte Subjekt eine weitere Menge an Ketonkörpern oder mittelkettigen Triglyceriden verbrauchen sollte.

12. Dialysefluid, umfassend Ketonkörper wie Acetoacetat, beta-Hydroxybutyrat oder pharmazeutisch verträgliche Derivate, Ester und Salze davon, zur Verwendung in einem Dialysetherapieverfahren zur Behandlung von Krebs.

13. Dialysefluid zur Verwendung in einem Dialysetherapieverfahren zur Behandlung von Krebs nach Anspruch 12, ferner umfassend mindestens eines von
a) Glutamin oder Glutamin enthaltenden Verbindungen; und
b) Glucose.

14. Dialysefluid zur Verwendung in einem Dialysetherapieverfahren zur Behandlung von Krebs nach Anspruch 12, wobei die Konzentration an:
a) Glutamin oder Glutamin enthaltenden Verbindungen 0 - 0,5 mM, vorzugsweise 0,05 - 0,3 mM, beträgt;
b) Glucose insgesamt 0 - 6 mM und vorzugsweise 0,5 - 4 mM ergibt; und
c) Ketonkörpern insgesamt 1 - 15 mM und vorzugsweise 2 - 12 mM betragen.

## Revendications

1. Système de traitement extracorporel du sang (50) pour traiter un sujet atteint d'un cancer, le système comprenant :
un circuit sanguin extracorporel (52a, 52b) ;
un circuit de fluide de dialysat (54a, 54b) ;
lesdits circuit sanguin extracorporel (52a, 52b) et circuit de fluide de dialysat (54a, 54b) étant divisés par une membrane (59) d'une unité de filtration (58) ;
au moins une pompe à sang (60) pour réguler l'écoulement de sang par l'intermédiaire du circuit sanguin (52a, 52b) ;
au moins une pompe à fluide de dialysat (62, 68) pour réguler l'écoulement de fluide de dialysat par l'intermédiaire du circuit de fluide de dialysat (54a, 54b) ;
éventuellement, une ou plusieurs lignes de perfusion (66, 80, 81, 82), chaque ligne de perfusion étant reliée au circuit sanguin extracorporel (52a, 52b) ou adaptée pour être directement reliée au système vasculaire du sujet à traiter, chaque ligne de perfusion comprenant une pompe à perfusion ;
une unité de calcul de système (64) reliée de manière opérationnelle à la pompe à sang (60) et à la pompe à fluide de dialysat (62, 68) et éventuellement à une ou
plusieurs pompes à perfusion d'une ou plusieurs lignes de perfusion (66, 80, 81, 82), ladite unité de calcul de système ayant une interface utilisateur comprenant un moyen d'entrée et un moyen d'affichage ;
l'unité de calcul de système (64) étant adaptée pour recevoir une valeur de concentration sanguine souhaitée GLN_{b} de glutamine (901) comprise entre 0,1 et 0,5 mM ;
l'unité de calcul de système (64) étant adaptée pour recevoir une valeur de concentration sanguine souhaitée GLUCOSE_{b} de glucose (903) comprise entre 2 et 4 mM ;
l'unité de calcul de système (64) étant adaptée pour recevoir une valeur de concentration sanguine souhaitée KETONE_{b} d'un corps cétonique, tel que l'acétoacétate, le bêta-hydroxybutyrate ou des dérivés, esters et sels pharmaceutiquement acceptables de ceux-ci (905), dans la plage de 1 à 15 mM ;
l'unité de calcul de système (64) étant adaptée pour recevoir une valeur de concentration GLN_{P} représentant la concentration de glutamine ou de composés contenant de la glutamine pharmaceutiquement acceptables dans le fluide de dialysat frais (902) ;
l'unité de calcul de système (64) étant adaptée pour recevoir une valeur de concentration GLUCOSEₚ représentant la concentration de glucose dans le fluide de dialysat frais (904) ;
éventuellement, l'unité de calcul de système (64) étant adaptée pour recevoir une valeur de concentration KETONEₚ, représentant la concentration d'un corps cétonique, tel que l'acétoacétate, le bêta-hydroxybutyrate ou des dérivés, esters et sels pharmaceutiquement acceptables de ceux-ci dans le fluide de dialysat frais (907) ;
éventuellement, l'unité de calcul de système (64) étant adaptée pour recevoir une valeur de concentration KETONEᵢ représentant la concentration d'un corps cétonique tel que l'acétoacétate, le bêta-hydroxybutyrate ou des dérivés, esters et sels pharmaceutiquement acceptables de ceux-ci dans un liquide de perfusion à perfuser dans la ligne sanguine extracorporelle (52b) ou directement dans le système vasculaire dudit sujet à traiter, par l'intermédiaire d'une desdites une ou plusieurs lignes de perfusion (66, 80, 81, 82) (906) ;
l'unité de calcul de système (64) étant adaptée pour recevoir une valeur de concentration réelle GLNₐ de glutamine dans le sang dudit sujet traité (909) et
recevoir une valeur de concentration réelle GLUCOSEₐ de glucose dans le sang dudit sujet traité (910) et recevoir une valeur de concentration réelle KETONEₐ d'un corps cétonique tel que l'acétoacétate et/ou le bêta-hydroxybutyrate (911) ;
l'unité de calcul de système (64) étant adaptée pour commander ladite pompe à sang (60) et ladite pompe à fluide de dialysat (62, 68) de manière à ce que la valeur de concentration sanguine réelle GLNₐ de glutamine soit amenée vers ou en dessous de GLN_{b} (912) et que la valeur de concentration sanguine réelle GLUCOSEₐ de glucose soit amenée vers ou en dessous de GLUCOSE_{b} (914) ;
et
si le système (50) comprend une ou plusieurs desdites lignes de perfusion (66, 80, 81, 82) et dans le cas où l'une desdites lignes de perfusion (66, 80, 81, 82) est adaptée pour perfuser ledit liquide de perfusion dans la ligne de sang extracorporelle (52b) ou directement dans le système vasculaire dudit sujet à traiter, l'unité de calcul de système (64) étant adaptée pour commander ladite pompe de perfusion de ladite ligne de perfusion de manière à ce que la valeur de concentration sanguine réelle de KETONEₐ soit amenée vers KETONE_{b} (920) ; ou en variante
si le système (50) ne comprend pas une telle ligne de perfusion (66, 80, 81, 82), l'unité de calcul de système (64) étant adaptée pour comparer KETONEₐ et KETONE_{b}, et si KETONEₐ < KETONE_{b}, afficher un message sur ledit dispositif d'affichage informant que le sujet traité doit consommer une quantité supplémentaire de corps cétoniques ou de triglycérides à chaîne moyenne.

2. Système de traitement extracorporel du sang (50) selon la revendication 1,
le système (50) comprenant une ou plusieurs desdites lignes de perfusion (66, 80, 81, 82), et
l'unité de calcul de système (64) étant adaptée pour recevoir une valeur de concentration KETONEᵢ représentant la concentration d'un corps cétonique tel que l'acétoacétate, le bêta-hydroxybutyrate ou des dérivés, esters et sels pharmaceutiquement acceptables de ceux-ci dans un liquide de perfusion à perfuser dans la ligne de sang extracorporel (52b) ou directement dans le système vasculaire du sujet à traiter, par l'intermédiaire de l'une desdites une ou plusieurs lignes de perfusion (66, 80, 81, 82) (906), et
l'unité de calcul de système (64) étant adaptée pour commander ladite pompe à perfusion de ladite ligne de perfusion de manière à ce que la valeur de concentration sanguine réelle KETONEₐ soit amenée vers KETONE_{b} (920).

3. Système de traitement extracorporel du sang (50) selon la revendication 2, l'unité de calcul de système (64) étant adaptée pour surveiller GLNₐ et initier la perfusion d'une composition contenant de la glutamine ou des composés contenant de la glutamine pharmaceutiquement acceptables si GLNₐ est inférieure à GLN_{b} (916) en démarrant une pompe à perfusion concernée dans l'une de la ou des lignes de perfusion (66, 80, 81, 82), et en maintenant ladite perfusion jusqu'à ce que GLNₐ soit égale à GLN_{b}.

4. Système de traitement extracorporel du sang (50) selon l'une quelconque des revendications 2 à 3, l'unité de calcul de système (64) étant adaptée pour surveiller GLUCOSEₐ, et initier la perfusion d'une composition contenant du glucose si GLUCOSEₐ est inférieure à GLUCOSE_{b} (918) en démarrant une pompe à perfusion pertinente dans l'une de la ou des lignes de perfusion (66, 80, 81, 82), et en maintenant ladite perfusion jusqu'à ce que GLUCOSEₐ soit égale à GLUCOSE_{b} (918).

5. Système selon l'une quelconque des revendications précédentes, la membrane de filtration ayant un seuil de poids moléculaire (MWCO) inférieur à 60 kDa.

6. Système selon la revendication 5, la membrane de filtration ayant un MWCO inférieur à environ 50 kDa ou inférieur à environ 40 kDa, inférieur à 30 kDa, inférieur à 10 kDa, inférieur à 5 kDa ou inférieur à 2 kDa.

7. Système selon l'une quelconque des revendications précédentes, le circuit sanguin comprenant un système de gestion thermique pour chauffer ou refroidir le sang dans la ligne sanguine pendant l'utilisation.

8. Système selon la revendication 7, le système de gestion thermique pouvant être contrôlé pour réguler la température du sang dans le circuit sanguin à une température comprise entre 20 °C et 43 °C.

9. Système selon n'importe quelle revendication précédente, comprenant en outre un ou plusieurs capteurs (S, 90, 91, 92, 93) pour la détection d'analytes choisis dans le groupe du glucose, de la glutamine et des corps cétoniques, le ou les capteurs (S, 90, 91, 92, 93) étant de préférence positionnés dans une partie d'effluent (54a) du circuit de fluide de dialysat, les capteurs étant en communication avec l'unité de traitement du système (64) et fournissant une sortie indicative de la concentration de l'analyte ou des analytes dans le sang ou plus préférablement dans le fluide de dialysat usé ; l'unité de traitement du système (64) étant configurée pour déterminer, à partir de la ou des sorties du ou des capteurs (S, 90, 91, 92, 93), une concentration sanguine représentative de l'analyte ou des analytes, surveillant ainsi au moins l'une de GLNₐ, GLUCOSEₐ et KETONEₐ.

10. Ensemble thérapeutique destiné à être utilisé dans un système de traitement extracorporel du sang (50) selon l'une quelconque des revendications 1 à 9, l'ensemble comprenant
une unité de filtration (58) comportant une membrane (59) qui divise une ligne de sang intégrée (52a, 52b) et une ligne de fluide de dialysat intégrée (54a, 54b), la ligne de sang (52a, 52b) et/ou la ligne de fluide de dialysat (54a, 54b) comportant des capteurs (S, 90, 91, 92, 93) pour surveiller au moins l'une de GLNₐ, GLUCOSEₐ et KETONEₐ.

11. Unité de calcul de système (64) adaptée pour commander un système de traitement extracorporel du sang (50) pour traiter un sujet atteint d'un cancer ; ladite unité de calcul de système comprenant :
une pluralité de moyens de sortie adaptés pour être reliés de manière opérationnelle à au moins une pompe à sang (60), au moins une pompe à fluide de dialysat (62, 68) et éventuellement une ou plusieurs pompes à perfusion pour réguler l'écoulement dans chacune d'une ou plusieurs lignes de perfusion (66, 80, 81, 82) ;
une interface utilisateur comprenant un moyen d'entrée et un moyen d'affichage ; et
un moyen de mémoire et un moyen de calcul ;
l'unité de calcul de système (64) étant adaptée pour recevoir une valeur de concentration sanguine souhaitée GLN_{b} de glutamine (901) ;
l'unité de calcul de système (64) étant adaptée pour recevoir une valeur de concentration sanguine souhaitée GLUCOSE_{b} de glucose (903) ;
l'unité de calcul de système (64) étant adaptée pour recevoir d'une valeur de concentration sanguine souhaitée KETONE_{b} d'un corps cétonique (905)
l'unité de calcul de système (64) étant adaptée pour recevoir une valeur de concentration du dialysat GLNₚ de glutamine (902) ;
l'unité de calcul de système (64) étant adaptée pour recevoir une valeur de concentration du dialysat GLUCOSEₚ de glucose (904) ;
éventuellement, l'unité de calcul de système (64) étant adaptée pour recevoir une valeur de concentration KETONEₚ, représentant la concentration d'un corps cétonique, tel que l'acétoacétate, le bêta-hydroxybutyrate ou des dérivés, esters et sels pharmaceutiquement acceptables de ceux-ci dans le fluide de dialysat frais (907) ;
éventuellement, l'unité de calcul de système (64) étant adaptée pour recevoir une valeur de concentration de liquide de perfusion KETONEᵢ d'un corps cétonique (906) ;
l'unité de calcul de système (64) étant adaptée pour recevoir une valeur de concentration sanguine réelle GLNₐ de glutamine (909) ;
l'unité de calcul de système (64) étant adaptée pour recevoir une valeur de concentration sanguine réelle GLUCOSEₐ de glucose (910) ;
l'unité de calcul de système (64) étant adaptée pour recevoir une valeur de concentration sanguine réelle KETONEₐ d'un corps cétonique tel que le bêta-hydroxybutyrate et/ou l'acétoacétate (911) ;
l'unité de calcul de système (64) étant adaptée pour commander ladite pompe à sang (60) et ladite pompe à fluide de dialysat (62, 68) de manière à ce que la valeur de concentration sanguine réelle GLNₐ de glutamine soit amenée vers ou en dessous de GLN_{b} (912), et que la valeur de concentration sanguine réelle GLUCOSEₐ de glucose soit amenée vers ou en dessous de Glucose_{b} (914) ; et
dans le cas où une pompe à perfusion est reliée de manière opérationnelle à l'unité de calcul de système (64), l'unité de calcul de système (64) étant adaptée pour commander la pompe à perfusion de manière à ce que la valeur de concentration sanguine réelle KETONEₐ soit amenée vers KETONE_{b} (920) ;
et
dans le cas où aucune pompe à perfusion n'est reliée de manière opérationnelle à l'unité de calcul de système (64), l'unité de calcul de système (64) étant adaptée pour comparer KETONEₐ et KETONE_{b}, et si KETONEₐ < KETONE_{b}, afficher un message sur ledit dispositif d'affichage informant que le sujet traité doit consommer une quantité supplémentaire de corps cétoniques ou de triglycérides à chaîne moyenne.

12. Fluide de dialyse, comprenant des corps cétoniques tels que l'acétoacétate, le bêta-hydroxybutyrate ou des dérivés, esters et sels pharmaceutiquement acceptables de ceux-ci, destiné à être utilisé dans un procédé de thérapie par dialyse pour traiter le cancer.

13. Fluide de dialyse destiné à être utilisé dans un procédé de dialyse thérapeutique pour traiter le cancer selon la revendication 12, comprenant en outre au moins l'un des éléments parmi
a) de la glutamine ou des composés contenant de la glutamine ; et
b) du glucose.

14. Fluide de dialyse destiné à être utilisé dans un procédé de dialyse thérapeutique traitant du cancer selon la revendication 12, la concentration de :
a) glutamine ou composés contenant de la glutamine s'élevant à 0 à 0,5 mM, et de préférence de 0,05 à 0,3 mM ;
b) de glucose s'élevant de 0 à 6 mM et de préférence de 0,5 à 4 mM ; et
c) corps cétoniques s'élevant de 1 à 15 mM et de préférence de 2 à 12 mM**.**
